# EUROPEAN PATENT APPLICATION

(11) **EP 4 678 188 A1**
(43) Date of publication of application: **14.01.2026**
(21) Application number: 24767208.2
(22) Date of filing: 07.03.2024
(51) Int. Cl.: A61K 45/00, A61K 31/496, A61P 25/00, A61P 25/14, A61P 25/18, A61P 43/00

(54) **PHARMACEUTICAL COMPOSITION FOR TREATING, PREVENTING, OR MANAGING BILIRUBIN-INDUCED NEUROLOGICAL DYSFUNCTION, PSYCHIATRIC DISORDERS ASSOCIATED WITH JAUNDICE, OR PSYCHIATRIC DISORDERS CAUSED BY BILIRUBIN UDP-GLUCURONOSYLTRANSFERASE GENE MUTATIONS**

(30) Priority: 09.03.2023 JP 2023036505
(71) Applicant: Ohnishi, Arata, Izumo-shi, Shimane 693--0001 (JP)
(72) Inventor: OHNISHI Arata, Izumo-shi, Shimane 693-0001 (JP)
(74) Representative: Sagittarius IP
(86) International application number: PCT/JP2024/008718
(87) International publication number: WO 2024/185842

(57) **Abstract**

The present inventors have found that administration of a 5-HT_{1A/1B} receptor agonist to a subject makes it possible to treat, prevent, or manage BIND, a psychiatric disorder associated with jaundice, or a psychiatric disorder caused by UGT1A1 gene mutation in the subject. Therefore, the present disclosure provides a pharmaceutical composition containing a 5-HT_{1A/1B} receptor agonist for use in treating, preventing, or managing BIND, a psychiatric disorder associated with jaundice, or a psychiatric disorder caused by UGT1A1 gene mutation.

## Description

### TECHNICAL FIELD

The present invention relates to pharmaceutical compositions and methods for treating, preventing, or managing bilirubin-induced neurological dysfunction (BIND) (bilirubin encephalopathy). The present invention also relates to pharmaceutical compositions and methods for treating, preventing, or managing psychiatric disorders associated with jaundice or psychiatric disorders caused by bilirubin UDP-glucuronosyltransferase (UGT1A1) gene mutations.

### BACKGROUND ART

Constitutional hyperbilirubinemia is a condition in which abnormality in UDP-glucuronosyltransferase (UGT1A1) in hepatocytes leads to inadequate processing of indirect bilirubin, resulting in elevated blood bilirubin levels and causing yellowing of the skin and eyes. Although constitutional hyperbilirubinemia is a well-known condition that affects 2% to 7% of the population, recent studies have revealed its association with psychiatric disorders. It has been suggested that mutations in UGT1A1 and the presence of jaundice may be among the contributing factors to schizophrenia and attention deficit hyperactivity disorder (ADHD). Currently, in Japan, it is estimated that 20% (over 200,000 individuals) of schizophrenia patients suffer from both constitutional hyperbilirubinemia and schizophrenia. However, patients with both jaundice and schizophrenia tend to suffer stronger extrapyramidal side effects caused by the dopamine receptor blocking effects of antipsychotic drugs compared to those without jaundice and schizophrenia, and their clinical symptoms are more severe both in the acute and stable phases, thereby making treatments of such patients more difficult (Non Patent Literatures 1 and 2).

BIND, also known as kernicterus, is a brain disorder caused by the neurotoxicity of elevated levels of unconjugated bilirubin in the blood, and can lead to intellectual disabilities and motor dysfunction. In recent years, an increase in bilirubin encephalopathy among premature babies has been observed, making it a major issue that urgently needs to be addressed in the field of neonatal medicine.

Regarding neurodegenerative diseases, therapeutic agents using serotonin receptor antagonists and the like have been under development. However, these therapeutic agents have been reported to have side effects including QT prolongation and limited efficacy, which remain unsolved (Patent Literatures 1-3 and Non-Patent Literatures 3-5). On the other hand, there have been very few studies aimed at treating psychiatric disorders or neurological dysfunction caused by elevated blood bilirubin levels, and no therapeutic agents have been developed for such conditions to date.

### CITATION LIST

### PATENT LITERATURE

Patent Literature 1: Japanese Translation of PCT International Application Publication No. JP-T-2008-500325
Patent Literature 2: Japanese Translation of PCT International Application Publication No. JP-T-2003-531829
Patent Literature 3: Japanese Translation of PCT International Application Publication No. JP-T-2006-516284

### NON PATENT LITERATURE

Non Patent Literature 1: Tsuyoshi Miyaoka, "Schizophrenia and idiopathic unconjugated hyperbilirubinemia (Gilbert's syndrome)", Psychiatria et neurologia Japonica (2011), 113, Volume 4, Pages 361-367
Non Patent Literature 2: Takahashi H. et al., "The role of extrastriatal dopamine D2 receptors in schizophrenia", Biological psychiatry Volume 59, Issue 10, May 2006 Pages 919-28
Non Patent Literature 3: Q Tang et al., "The 5-HT2 antagonist ketanserin is an open channel blocker of human cardiac ether-a-go-go-related gene (hERG) potassium channels", British Journal of Pharmacology (2008) 155, 365-3373
Non Patent Literature 4: Cummings J. et al., "Pimavanserin for patients with Parkinson's disease psychosis: a randomised, placebo-controlled phase 3 trial", Lancet 2014; 383: 533-40
Non Patent Literature 5: McFarland K. et al., "Pimavanserin, a 5-HT2A inverse agonist, reverses psychosis-like behaviors in a rodent model of Parkinson's disease", Behav Pharmacol. 2011 Oct;22(7):681-92
Non Patent Literature 6: Hayashida M. et al., "Hyperbilirubinemia-related behavioral and neuropathological changes in rats: A possible schizophrenia animal model", Progress in Neuropsychopharmacology & Biological Psychiatry, 33 (2009) 581-588
Non Patent Literature 7: Tsuchie K. et al., "The effects of antipsychotics on behavioral abnormalities of the Gunn rat (unconjugated hyperbilirubinemia rat), a rat model of schizophrenia", Asian Journal of Psychiatry, Volume 6, Issue 2, April 2013, Pages 119-123
Non Patent Literature 8: Takeuchi K. et al., "Genetic polymorphisms of bilirubin uridine diphosphate-glucuronosyltransferase gene in Japanese patients with Crigler-Najjar syndrome or Gilbert's syndrome as well as in healthy Japanese subjects", Journal of Gastroenterology and Hepatology (2004) 19, 1023-1028
Non Patent Literature 9: Kimura K. et al., "Structures of the 5-HT2A receptor in complex with the antipsychotics risperidone and zotepine", Nature Structural & Molecular Biology, volume 26, pages 121-128 (2019)

### SUMMARY OF INVENTION

### TECHNICAL PROBLEM

An object of the present disclosure is to provide pharmaceutical compositions and methods for treating, preventing, or managing BIND, psychiatric disorders associated with jaundice, or psychiatric disorders caused by UGT1A1 gene mutations.

### SOLUTION TO PROBLEM

In developing therapeutic agents for psychiatric disorders, the present inventors focused on a jaundice model rat (Gunn rat) carrying a UGT1A1 gene mutation. The Gunn rat is known to exhibit cognitive and behavioral impairments associated with schizophrenia (Non-Patent Literatures 6 and 7). The Gunn rat may serve as a model animal for BIND, psychiatric disorders associated with jaundice, or psychiatric disorders caused by UGT1A1 gene mutations. The present inventors discovered that administration of 5-HT_{1A/1B} receptor agonist to Gunn rats dramatically improved their cognitive and behavioral impairments, which led to the development of the present invention.

Accordingly, the present disclosure provides inventions in the following embodiments.

### (Embodiment 1)

A pharmaceutical composition for use in treating, preventing, or managing a bilirubin-induced neurological dysfunction, a psychiatric disorder associated with jaundice, or a psychiatric disorder caused by bilirubin UDP-glucuronosyltransferase gene mutation, wherein the pharmaceutical composition comprises a 5-HT_{1A/1B} receptor agonist.

### (Embodiment 2)

A pharmaceutical composition for use in treating, preventing, or managing a bilirubin-induced neurological dysfunction in a subject, wherein the pharmaceutical composition comprises a 5-HT_{1A/1B} receptor agonist, wherein the subject has a high bilirubin level in his/her biological sample compared to a bilirubin level of a healthy subject, or the subject has jaundice.

### (Embodiment 3)

A pharmaceutical composition for use in treating, preventing, or managing a bilirubin-induced neurological dysfunction in a subject, wherein the pharmaceutical composition comprises a 5-HT_{1A/1B} receptor agonist, wherein the subject has a bilirubin UDP-glucuronosyltransferase gene mutation.

### (Embodiment 4)

A pharmaceutical composition for use in a method for treating, preventing, or managing a bilirubin-induced neurological dysfunction in a subject, wherein the pharmaceutical composition comprises 5-HT_{1A/1B} receptor agonist,
wherein the method comprises steps of:
(a) obtaining a biological sample from the subject;
(b) measuring a bilirubin level in the biological sample obtained in the step (a); and
(c) administering to the subject a 5-HT_{1A/1B} receptor agonist when the bilirubin level measured in the step (b) is higher than a bilirubin level of a healthy subject, or when it is determined that the subject has jaundice based on the bilirubin level measured in the step (b).

### (Embodiment 5)

A pharmaceutical composition for use in a method for treating, preventing, or managing a bilirubin-induced neurological dysfunction in a subject, wherein the pharmaceutical composition comprises a 5-HT_{1A/1B} receptor agonist,
wherein the method comprises steps of:
(a) determining whether the subject has a bilirubin UDP-glucuronosyltransferase gene mutation; and
(b) administering to the subject a 5-HT_{1A/1B} receptor agonist when it is determined that the subject has a bilirubin UDP-glucuronosyltransferase gene mutation in the step (a).

### (Embodiment 6)

A pharmaceutical composition for use in treating, preventing, or managing a psychiatric disorder in a subject, wherein the pharmaceutical composition comprises a 5-HT_{1A/1B} receptor agonist, wherein the subject has a high bilirubin level in his/her biological sample compared to a bilirubin level of a healthy subject, or the subject has jaundice.

### (Embodiment 7)

A pharmaceutical composition for use in treating, preventing, or managing a psychiatric disorder in a subject, wherein the pharmaceutical composition comprises a 5-HT_{1A/1B} receptor agonist, wherein the subject has a bilirubin UDP-glucuronosyltransferase gene mutation.

### (Embodiment 8)

A pharmaceutical composition for use in a method for treating, preventing, or managing a psychiatric disorder in a subject, wherein the pharmaceutical composition comprises a 5-HT_{1A/1B} receptor agonist,
wherein the method comprises steps of:
(a) obtaining a biological sample from the subject;
(b) measuring a bilirubin level in the biological sample obtained in the step (a); and
(c) administering to the subject a 5-HT_{1A/1B} receptor agonist when the bilirubin level measured in the step (b) is higher than a bilirubin level of a healthy subject, or when it is determined that the subject has jaundice based on the bilirubin level measured in the step (b).

### (Embodiment 9)

A pharmaceutical composition for use in a method for treating, preventing, or managing a psychiatric disorder in a subject, wherein the pharmaceutical composition comprises a 5-HT_{1A/1B} receptor agonist,
wherein the method comprises steps of:
(a) determining whether the subject has a bilirubin UDP-glucuronosyltransferase gene mutation; and
(b) administering to the subject a 5-HT_{1A/1B} receptor agonist when it is determined that the subject has a bilirubin UDP-glucuronosyltransferase gene mutation in the step (a).

### (Embodiment 10)

The pharmaceutical composition according to any one of Embodiments 1 to 9, wherein the 5-HT_{1A/1B} receptor agonist is a compound of the following formula (I), or a pharmaceutically acceptable salt, solvate, hydrate, stereoisomer, tautomer, or a deuterium-labeled compound thereof, 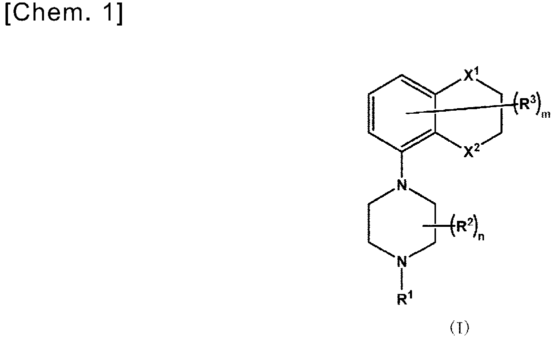
wherein R¹ is hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
R² and R³ are each independently, at each occurrence, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
X¹ and X² are each independently oxygen, sulfur, or -CR^{a}R^{b};
R^{a} and R^{b} are each independently hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
n is an integer of 0 to 4; and
m is an integer of 0 to 5.

### (Embodiment 11)

The pharmaceutical composition according to Embodiment 10, wherein, in the compound of formula (I), X¹ and X² are oxygen.

### (Embodiment 12)

The pharmaceutical composition according to Embodiment 10 or 11, wherein, in the compound of formula (I), R¹ is hydrogen.

### (Embodiment 13)

The pharmaceutical composition according to any one of Embodiments 10 to 12, wherein, in the compound of formula (I), n is 0.

### (Embodiment 14)

The pharmaceutical composition according to any one of Embodiments 10 to 13, wherein, in the compound of formula (I), m is 0.

### (Embodiment 15)

The pharmaceutical composition according to any one of Embodiments 10 to 14, wherein, in the compound of formula (I), X¹ and X² are oxygen, R¹ is hydrogen, n is 0, and m is 0, or the compound of formula (I) is eltoprazine.

### (Embodiment 16)

The pharmaceutical composition according to any one of Embodiments 1 to 9, wherein the psychiatric disorder is schizophrenia or attention deficit hyperactivity disorder.

### (Embodiment 17)

A kit for use in treating, preventing, or managing a bilirubin-induced neurological dysfunction, a psychiatric disorder associated with jaundice, or a psychiatric disorder caused by bilirubin UDP-glucuronosyltransferase gene mutation wherein the kit comprises a 5-HT_{1A/1B} receptor agonist or the pharmaceutical composition according to any one of Embodiments 1 to 16.

### (Embodiment 18)

The kit according to Embodiment 17, further comprising a reagent for measuring a bilirubin level in a biological sample and/or a reagent for measuring bilirubin UDP-glucuronosyltransferase gene mutation.

Further, the present disclosure provides inventions in the following embodiments.

### (Embodiment 19)

A method for treating, preventing, or managing a bilirubin-induced neurological dysfunction, a psychiatric disorder associated with jaundice, or a psychiatric disorder caused by bilirubin UDP-glucuronosyltransferase gene mutation in a subject in need thereof, wherein the method comprises a step of administering a therapeutically effective amount of a 5-HT_{1A/1B} receptor agonist to the subject.

### (Embodiment 20)

A method for treating, preventing, or managing a bilirubin-induced neurological dysfunction in a subject in need thereof, wherein the subject has a high bilirubin level in his/her biological sample compared to a bilirubin level of a healthy subject, or the subject has jaundice, wherein the method comprises a step of administering a therapeutically effective amount of a 5-HT_{1A/1B} receptor agonist to the subject.

### (Embodiment 21)

A method for treating, preventing, or managing a bilirubin-induced neurological dysfunction in a subject in need thereof, wherein the subject has a bilirubin UDP-glucuronosyltransferase gene mutation, wherein the method comprises a step of administering a therapeutically effective amount of a 5-HT_{1A/1B} receptor agonist to the subject.

### (Embodiment 22)

A method for treating, preventing, or managing a bilirubin-induced neurological dysfunction in a subject in need thereof,
wherein the method comprises steps of:
(a) obtaining a biological sample from the subject;
(b) measuring a bilirubin level in the biological sample obtained in the step (a); and
(c) administering to the subject a therapeutically effective amount of a 5-HT_{1A/1B} receptor agonist when the bilirubin level measured in the step (b) is higher than a bilirubin level of a healthy subject, or when it is determined that the subject has jaundice based on the bilirubin level measured in the step (b).

### (Embodiment 23)

A method for treating, preventing, or managing a bilirubin-induced neurological dysfunction in a subject in need thereof,
wherein the method comprises steps of:
(a) determining whether the subject has a bilirubin UDP-glucuronosyltransferase gene mutation; and
(b) administering to the subject a therapeutically effective amount of a 5-HT_{1A/1B} receptor agonist when it is determined that the subject has a bilirubin UDP-glucuronosyltransferase gene mutation in the step (a).

### (Embodiment 24)

A method for treating, preventing, or managing a psychiatric disorder in a subject in need thereof, wherein the subject has a high bilirubin level in his/her biological sample compared to a bilirubin level of a healthy subject, or the subject has jaundice, wherein the method comprises a step of administering a therapeutically effective amount of a 5-HT_{1A/1B} receptor agonist to the subject.

### (Embodiment 25)

A method for treating, preventing, or managing a psychiatric disorder in a subject in need thereof, wherein the subject has a bilirubin UDP-glucuronosyltransferase gene mutation, wherein the method comprises a step of administering a therapeutically effective amount of a 5-HT_{1A/1B} receptor agonist to the subject.

### (Embodiment 26)

A method for treating, preventing, or managing a psychiatric disorder in a subject in need thereof,
wherein the method comprises steps of:
(a) obtaining a biological sample from the subject;
(b) measuring a bilirubin level in the biological sample obtained in the step (a); and
(c) administering to the subject a therapeutically effective amount of a 5-HT_{1A/1B} receptor agonist when the bilirubin level measured in the step (b) is higher than a bilirubin level of a healthy subject, or when it is determined that the subject has jaundice based on the bilirubin level measured in the step (b).

### (Embodiment 27)

A method for treating, preventing, or managing a psychiatric disorder in a subject in need thereof,
wherein the method comprises steps of:
(a) determining whether the subject has a bilirubin UDP-glucuronosyltransferase gene mutation; and
(b) administering to the subject a therapeutically effective amount of a 5-HT_{1A/1B} receptor agonist when it is determined that the subject has a bilirubin UDP-glucuronosyltransferase gene mutation in the step (a).

### (Embodiment 28)

The method according to any one of Embodiments 19 to 27, wherein the 5-HT_{1A/1B} receptor agonist is a compound of the following formula (I), or a pharmaceutically acceptable salt, solvate, hydrate, stereoisomer, tautomer, or a deuterium-labeled compound thereof, 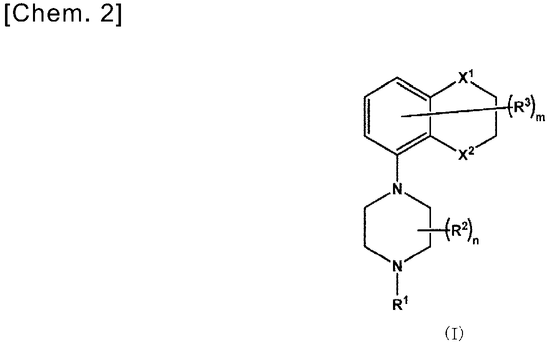
wherein R¹ is hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
R² and R³ are each independently, at each occurrence, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
X¹ and X² are each independently oxygen, sulfur, or -CR^{a}R^{b};
R^{a} and R^{b} are each independently hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
n is an integer of 0 to 4; and
m is an integer of 0 to 5.

### (Embodiment 29)

The method according to Embodiment 28, wherein, in the compound of formula (I), X¹ and X² are oxygen.

### (Embodiment 30)

The method according to Embodiment 28 or 29, wherein, in the compound of formula (I), R¹ is hydrogen.

### (Embodiment 31)

The method according to any one of Embodiments 28 to 30, wherein, in the compound of formula (I), n is 0.

### (Embodiment 32)

The method according to any one of Embodiments 28 to 31, wherein, in the compound of formula (I), m is 0.

### (Embodiment 33)

The method according to any one of Embodiments 28 to 32, wherein, in the compound of formula (I), X¹ and X² are oxygen, R¹ is hydrogen, n is 0, and m is 0, or the compound of formula (I) is eltoprazine.

### (Embodiment 34)

The method according to any one of Embodiments 19 to 33, wherein the psychiatric disorder is schizophrenia or attention deficit hyperactivity disorder.

### (Embodiment 35)

A kit for treating, preventing, or managing a bilirubin-induced neurological dysfunction, a psychiatric disorder associated with jaundice, or a psychiatric disorder caused by bilirubin UDP-glucuronosyltransferase gene mutation in a subject in need thereof, wherein the kit comprises a therapeutically effective amount of a 5-HT_{1A/1B} receptor agonist or the pharmaceutical composition according to any one of Embodiments 1 to 16, and instructions for treating, preventing, or managing a bilirubin-induced neurological dysfunction, a psychiatric disorder associated with jaundice, or a psychiatric disorder caused by bilirubin UDP-glucuronosyltransferase gene mutation.

### (Embodiment 36)

The kit according to Embodiment 35, further comprising a reagent for measuring a bilirubin level in a biological sample of the subject and/or a reagent for measuring bilirubin UDP-glucuronosyltransferase gene mutation.

Furthermore, the present disclosure provides inventions in the following embodiments.

### (Embodiment 37)

A 5-HT_{1A/1B} receptor agonist for use in treating, preventing, or managing a bilirubin-induced neurological dysfunction, a psychiatric disorder associated with jaundice, or a psychiatric disorder caused by bilirubin UDP-glucuronosyltransferase gene mutation.

### (Embodiment 38)

A 5-HT_{1A/1B} receptor agonist for use in treating, preventing, or managing a bilirubin-induced neurological dysfunction in a subject, wherein the subject has a high bilirubin level in his/her biological sample compared to a bilirubin level of a healthy subject, or the subject has jaundice.

### (Embodiment 39)

A 5-HT_{1A/1B} receptor agonist for use in treating, preventing, or managing a bilirubin-induced neurological dysfunction in a subject, wherein the subject has a bilirubin UDP-glucuronosyltransferase gene mutation.

### (Embodiment 40)

A 5-HT_{1A/1B} receptor agonist for use in a method for treating, preventing, or managing a bilirubin-induced neurological dysfunction in a subject,
wherein the method comprises steps of:
(a) obtaining a biological sample from the subject;
(b) measuring a bilirubin level in the biological sample obtained in the step (a); and
(c) administering to the subject a 5-HT_{1A/1B} receptor agonist when the bilirubin level measured in the step (b) is higher than a bilirubin level of a healthy subject, or when it is determined that the subject has jaundice based on the bilirubin level measured in the step (b).

### (Embodiment 41)

A 5-HT_{1A/1B} receptor agonist for use in a method for treating, preventing, or managing a bilirubin-induced neurological dysfunction in a subject,
wherein the method comprises steps of:
(a) determining whether the subject has a bilirubin UDP-glucuronosyltransferase gene mutation; and
(b) administering to the subject a 5-HT_{1A/1B} receptor agonist when it is determined that the subject has a bilirubin UDP-glucuronosyltransferase gene mutation in the step (a).

### (Embodiment 42)

A 5-HT_{1A/1B} receptor agonist for use in treating, preventing, or managing a psychiatric disorder in a subject, wherein the subject has a high bilirubin level in his/her biological sample compared to a bilirubin level of a healthy subject, or the subject has jaundice.

### (Embodiment 43)

A 5-HT_{1A/1B} receptor agonist for use in treating, preventing, or managing a psychiatric disorder in a subject, wherein the subject has a bilirubin UDP-glucuronosyltransferase gene mutation.

### (Embodiment 44)

A 5-HT_{1A/1B} receptor agonist for use in a method for treating, preventing, or managing a psychiatric disorder in a subject,
wherein the method comprises steps of:
(a) obtaining a biological sample from the subject;
(b) measuring a bilirubin level in the biological sample obtained in the step (a); and
(c) administering to the subject a 5-HT_{1A/1B} receptor agonist when the bilirubin level measured in the step (b) is higher than a bilirubin level of a healthy subject, or when it is determined that the subject has jaundice based on the bilirubin level measured in the step (b).

### (Embodiment 45)

A 5-HT_{1A/1B} receptor agonist for use in a method for treating, preventing, or managing a psychiatric disorder in a subject,
wherein the method comprises steps of:
(a) determining whether the subject has a bilirubin UDP-glucuronosyltransferase gene mutation; and
(b) administering to the subject a 5-HT_{1A/1B} receptor agonist when it is determined that the subject has a bilirubin UDP-glucuronosyltransferase gene mutation in the step (a).

### (Embodiment 46)

The 5-HT_{1A/1B} receptor agonist for use according to any one of Embodiments 37 to 45, wherein the 5-HT_{1A/1B} receptor agonist is a compound of the following formula (I), or a pharmaceutically acceptable salt, solvate, hydrate, stereoisomer, tautomer, or a deuterium-labeled compound thereof, 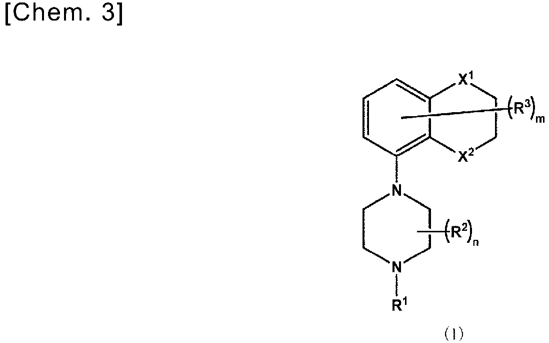
wherein R¹ is hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
R² and R³ are each independently, at each occurrence, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
X¹ and X² are each independently oxygen, sulfur, or -CR^{a}R^{b};
R^{a} and R^{b} are each independently hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
n is an integer of 0 to 4; and
m is an integer of 0 to 5.

### (Embodiment 47)

The 5-HT_{1A/1B} receptor agonist for use according to Embodiment 46, wherein, in the compound of formula (I), X¹ and X² are oxygen.

### (Embodiment 48)

The 5-HT_{1A/1B} receptor agonist for use according to Embodiment 46 or 47, wherein, in the compound of formula (I), R¹ is hydrogen.

### (Embodiment 49)

The 5-HT_{1A/1B} receptor agonist for use according to any one of Embodiments 46 to 48, wherein, in the compound of formula (I), n is 0.

### (Embodiment 50)

The 5-HT_{1A/1B} receptor agonist for use according to any one of Embodiments 46 to 49, wherein, in the compound of formula (I), m is 0.

### (Embodiment 51)

The 5-HT_{1A/1B} receptor agonist for use according to any one of Embodiments 46 to 49, wherein, in the compound of formula (I), X¹ and X² are oxygen, R¹ is hydrogen, n is 0, and m is 0, or the compound of formula (I) is eltoprazine.

### (Embodiment 52)

The 5-HT_{1A/1B} receptor agonist for use according to any one of Embodiments 37 to 51, wherein the psychiatric disorder is schizophrenia or attention deficit hyperactivity disorder.

### (Embodiment 53)

A kit for treating, preventing, or managing a bilirubin-induced neurological dysfunction, a psychiatric disorder associated with jaundice, or a psychiatric disorder caused by bilirubin UDP-glucuronosyltransferase gene mutation in a subject, wherein the kit comprises a 5-HT_{1A/1B} receptor agonist or a therapeutically effective amount of the 5-HT_{1A/1B} receptor agonist according to any one of Embodiments 37 to 52, and instructions for treating, preventing, or managing a bilirubin-induced neurological dysfunction, a psychiatric disorder associated with jaundice, or a psychiatric disorder caused by bilirubin UDP-glucuronosyltransferase gene mutation.

### (Embodiment 54)

The kit according to Embodiment 53, further comprising a reagent for measuring a bilirubin level in a biological sample of the subject and/or a reagent for measuring bilirubin UDP-glucuronosyltransferase gene mutation.

### ADVANTAGEOUS EFFECTS OF INVENTION

The present disclosure makes it possible to treat, prevent, or manage BIND, psychiatric disorders associated with jaundice, or psychiatric disorders caused by UGT1A1 gene mutations.

### BRIEF DESCRIPTION OF DRAWINGS

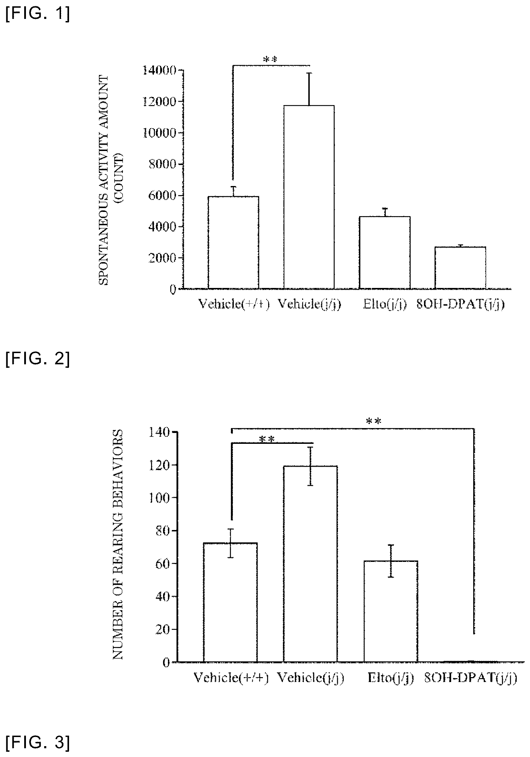

FIG. 1 shows a spontaneous activity amount in an open field test of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of 8OH-DPAT (8OH-DPAT(j/j)). In the figure, ** indicates p < 0.01 (Dunnett's test comparing each group with the Vehicle(+/+) group).
FIG. 2 shows the number of rearing behaviors in an open field test of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of 8OH-DPAT (8OH-DPAT(j/j)). In the figure, ** indicates p < 0.01 (Dunnett's test comparing each group with the Vehicle(+/+) group).
FIG. 3 shows the number of grooming behaviors in an open field test of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of 8OH-DPAT (8OH-DPAT(j/j)). In the figure, ** indicates p < 0.01 (Dunnett's test comparing each group with the Vehicle(+/+) group).

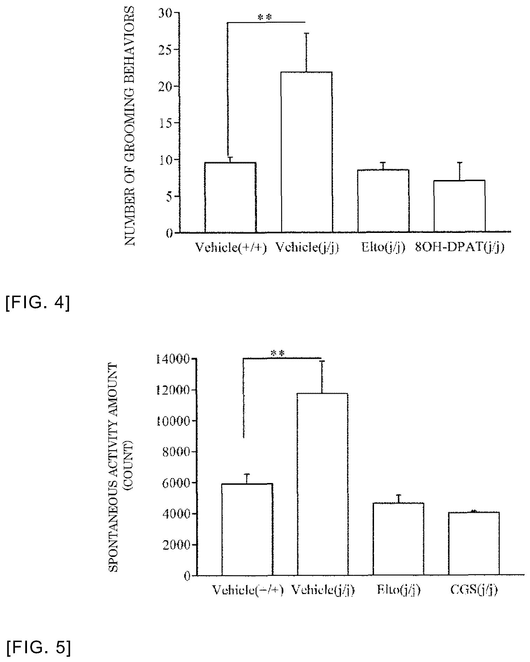

FIG. 4 shows a spontaneous activity amount in an open field test of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of CGS12066A (CGS(j/j)). In the figure, ** indicates p < 0.01 (Dunnett's test comparing each group with the Vehicle(+/+) group).
FIG. 5 shows the number of rearing behaviors in an open field test of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of CGS12066A (CGS(j/j)). In the figure, ** indicates p < 0.01 (Dunnett's test comparing each group with the Vehicle(+/+) group).

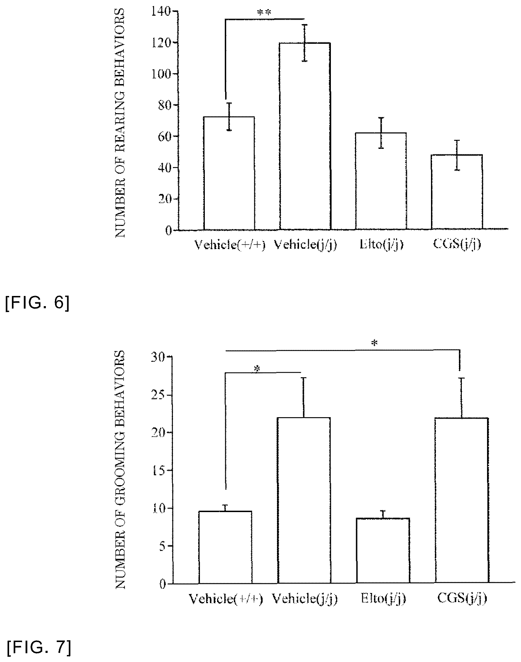

FIG. 6 shows the number of grooming behaviors in an open field test of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of CGS12066A (CGS(j/j)). In the figure, * indicates p < 0.05 (Dunnett's test comparing each group with the Vehicle(+/+) group).
FIG. 7 shows the number of following behaviors in a social interaction test of the following groups: Wistar rats administered only with a solvent (Vehicle(+/+)), Gunn rats administered only with a solvent (Vehicle(j/j)), Gunn rats administered with 0.3 mg/kg of eltoprazine (Elto 0.3mg(j/j)), and Gunn rats administered with 1 mg/kg of eltoprazine (Elto 1mg(j/j)). In the figure, *** indicates p < 0.001 (Dunnett's test comparing each group with the Vehicle(+/+) group).

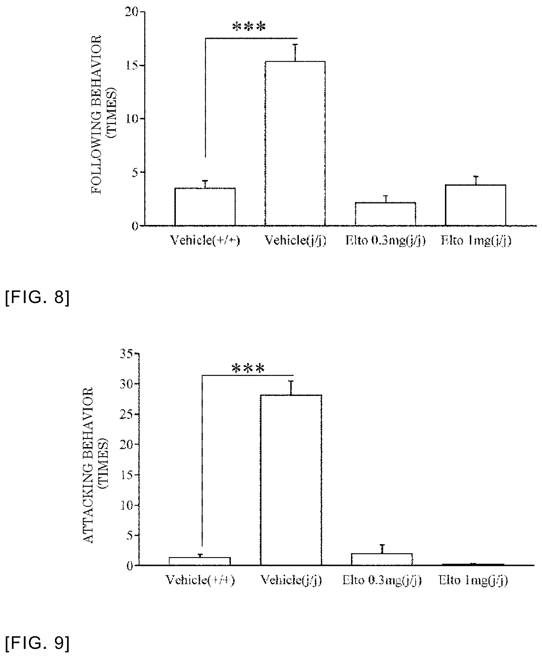

FIG. 8 shows the number of attacking behaviors in a social interaction test of the following groups: Wistar rats administered only with a solvent (Vehicle(+/+)), Gunn rats administered only with a solvent (Vehicle(j/j)), Gunn rats administered with 0.3 mg/kg of eltoprazine (Elto 0.3mg(j/j)), and Gunn rats administered with 1 mg/kg of eltoprazine (Elto 1mg(j/j)). In the figure, *** indicates p < 0.001 (Dunnett's test comparing each group with the Vehicle(+/+) group).
FIG. 9 shows the duration of sniffing or grooming the partner in a social interaction test of the following groups: Wistar rats administered only with a solvent (Vehicle(+/+)), Gunn rats administered only with a solvent (Vehicle(j/j)), Gunn rats administered with 0.3 mg/kg of eltoprazine (Elto 0.3mg(j/j)), and Gunn rats administered with 1 mg/kg of eltoprazine (Elto 1mg(j/j)). In the figure, *** indicates p < 0.001 (Dunnett's test comparing each group with the Vehicle(+/+) group).

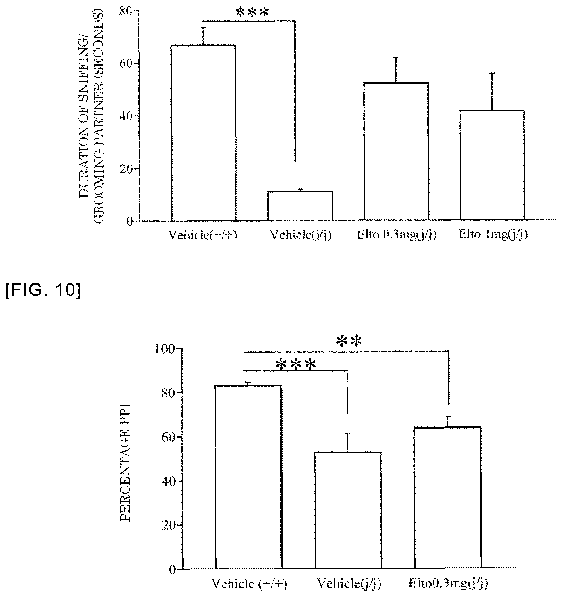

FIG. 10 shows the results of percentage PPI for Wistar rats administered only with a solvent (Vehicle(+/+)), Gunn rats administered only with a solvent (Vehicle(j/j)), and Gunn rats administered with 0.3 mg/kg of eltoprazine (Elto 0.3mg(j/j)). In the figure, ** indicates p < 0.01, and *** indicates p < 0.001 (Dunnett's test comparing each group with the Vehicle(+/+) group).

### DESCRIPTION OF EMBODIMENTS

### (1. Definition)

The term "psychiatric disorder" as used in the present specification has the broadest meaning as used in the technical field of the present disclosure. In general, a psychiatric disorder is a superordinate concept that encompasses a variety of symptoms that may cause significant distress or functional impairment due to a mental state that deviates from the average or normative values. Examples of the term "psychiatric disorder" include, but are not limited to, based on the diagnostic and statistical manual classification according to the DSM-IV (Diagnostic and Statistical Manual of Mental Disorders), delirium, dementia, amnestic disorders, other cognitive disorders, mental dysfunctions due to general medical conditions, substance-related disorders, schizophrenia and other psychotic disorders, mood disorders, anxiety disorders, somatoform disorders, factitious disorders, dissociative disorders, sexual disorders and gender identity disorders, eating disorders, sleep disorders, impulse-control disorders not elsewhere classified, adjustment disorders, and personality disorders. Other examples include attention deficit hyperactivity disorder (ADHD), autism, Alzheimer's disease, cognitive impairment, depression, bipolar disorder (manic-depressive illness), neurodevelopmental disorders, cognitive dysfunction caused by neurological disorder due to infection during pregnancy, mental disorders caused by immune dysfunction, epilepsy, organic mental disorders, toxic psychosis, intellectual disabilities (mental retardation), psychopathy, neurosis, syphilitic psychosis, senile psychosis, cerebrovascular psychosis, psychosis due to head trauma, atypical endogenous psychosis, endocrine psychosis, exogenous reactive type, and involutional psychosis.

The term "Bilirubin-Induced Neurological Dysfunction" (BIND) as used in the present specification has the broadest meaning as used in the technical field of the present disclosure and generally refers to brain dysfunction caused by the neurotoxicity of bilirubin. In this specification, "bilirubin-induced neurological dysfunction" is also referred to as BIND. In particular, indirect bilirubin (unbound bilirubin; UB-Bil) is neurotoxic and selectively impairs regions such as the globus pallidus, subthalamic nucleus, hippocampus, oculomotor nucleus, ventral cochlear nucleus, cerebellar Purkinje cells, and cerebellar dentate nucleus. In premature babies, even mild hyperbilirubinemia is known to cause BIND. "Bilirubin-induced neurological dysfunction" is also referred to as "kernicterus" or "bilirubin encephalopathy."

The term "bilirubin UDP-glucuronosyltransferase" as used in the present specification refers to an enzyme that converts endogenous unconjugated bilirubin (indirect bilirubin) into conjugated bilirubin (direct bilirubin). In this specification, bilirubin UDP-glucuronosyltransferase is also referred to as UGT1A1.

The term "bilirubin UDP-glucuronosyltransferase gene" or "UGT1A1 gene" as used in the present specification refers to the gene encoding bilirubin UDP-glucuronosyltransferase (UGT1A1). Mutations in this gene are known to be a potential cause of constitutional hyperbilirubinemia.

The term "bilirubin" as used in the present specification has the broadest meaning as used in the technical field of the present disclosure, and generally refers to a normal yellow catabolite of heme. The term "bilirubin" as used in the present specification includes total bilirubin, indirect bilirubin, and direct bilirubin. Indirect bilirubin refers to bilirubin before it is transported to the liver, and direct bilirubin refers to bilirubin after it has been processed in the liver. Total bilirubin is a collective term for indirect and direct bilirubin.

The term "gene mutation" as used in the present specification has the broadest meaning as used in the technical field of the present disclosure, and generally refers to high-frequency abnormalities (such as deletion, substitution, or insertion of bases) in a gene, which leads to reduced or loss of gene function.

The term "jaundice" as used in the present specification has the broadest meaning as used in the technical field of the present disclosure, and generally refers to a condition in which bile pigments (bilirubin) abnormally increase in the blood or tissues, causing yellowing of the skin, mucous membranes, and other tissues. In particular, "Jaundice" refers to cases in which the unconjugated bilirubin level in the blood or urine is elevated. "Jaundice" includes latent jaundice (e.g., blood bilirubin level of about 1 mg/dl to about 2 mg/dl), mild jaundice (e.g., blood bilirubin level of about 2 mg/dl to about 10 mg/dl), moderate jaundice (e.g., blood bilirubin level of about 10 mg/dl to about 20 mg/dl), and severe jaundice (e.g., blood bilirubin level of about 20 mg/dl or more). Specific examples of "jaundice" include, but are not limited to, physiological jaundice and pathological jaundice, such as direct bilirubin-predominant jaundice, obstructive jaundice, hepatocellular jaundice, indirect bilirubin-predominant jaundice, hemolytic jaundice, constitutional hyperbilirubinemia, and neonatal jaundice.

The term "constitutional hyperbilirubinemia" as used in the present specification has the broadest meaning as used in the technical field of the present disclosure, and generally refers to a condition in which there is a congenital defect in the metabolism of bilirubin. "Constitutional hyperbilirubinemia" is associated with elevated levels of unconjugated bilirubin particularly in the blood or urine. Examples of "constitutional hyperbilirubinemia" include Gilbert syndrome, Crigler-Najjar syndrome, Dubin-Johnson syndrome, Rotor syndrome, and the like.

The term "5-HT_{1A/1B} receptor" as used in the present specification has the broadest meaning as used in the technical field of the present disclosure, and generally refers to both the 5-HT_{1A} receptor and the 5-HT_{1B} receptor, which are subtypes of serotonin (5-HT) receptors.

The term "agonist" as used in the present specification has the broadest meaning as used in the technical field of the present disclosure, and generally refers to a compound that enhances the activity of a receptor upon contact with the receptor.

The term "antagonist" as used in the present specification has the broadest meaning as used in the technical field of the present disclosure, and generally refers to a compound that acts competitively with an agonist or inverse agonist in binding to a receptor, thereby blocking the action of the agonist or inverse agonist at the receptor. However, an antagonist (also known as a "neutral" antagonist) does not affect the constitutive activity.

The term "5-HT_{1A/1B} receptor agonist" as used in the present specification refers to both 5-HT_{1A} receptor agonist and 5-HT_{1B} receptor agonist.

The term "biological sample" as used in the present specification has the broadest meaning as used in the technical field of the present invention. For example, the term "biological sample" refers to bodily fluids such as blood or urine. The term "blood" includes serum, plasma, and whole blood.

The term "brain" as used in the present specification has the broadest meaning as used in the technical field of the present disclosure. The brain refers to the brain of a subject, such as a human brain. Specific examples of the brain include, but are not limited to, the cerebrum, cerebral cortex, diencephalon, cerebellum, and brainstem. More specific examples of the brain include, but are not limited to, the parietal lobe, frontal lobe, temporal lobe, occipital lobe, midbrain, striatum, hippocampus, nucleus originis, raphe nuclei (e.g., dorsal raphe nucleus, nucleus raphe magnus, and lateral part of the dorsal raphe nucleus), forebrain, medulla oblongata, pons, thalamus, hypothalamus, pituitary gland, globus pallidus, oculomotor nucleus, ventral cochlear nucleus, cerebellar Purkinje cells, cerebellar dentate nucleus, and basal ganglia.

The term "alkyl" as used in the present specification refers to a monovalent group resulting from the loss of one hydrogen atom from an aliphatic saturated hydrocarbon. For example, the "alkyl" has 1 to 10, 1 to 8, 1 to 6, 1 to 5, 1 to 4, 1 to 3, 1 to 2, or 2 to 6 carbon atoms. The "alkyl" may be linear or branched. Examples of "alkyl groups" include, but are not limited to, methyl, ethyl, propyl, isopropyl, 2,2-dimethylethyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, methylbutyl, pentyl, isopentyl, neopentyl, n-hexyl, n-heptyl, n-octyl, n-nonyl, and n-decyl.

The term "alkenyl" as used in the present specification refers to a monovalent group resulting from the loss of one hydrogen atom from an aliphatic unsaturated hydrocarbon having at least one double bond. For example, "alkenyl" has 2 to 10, 2 to 8, 2 to 6, 2 to 5, 2 to 4, 2 to 3, 3 to 6, 3 to 8, 4 to 6, 4 to 7, or 4 to 8 carbon atoms. The alkenyl group may be linear or branched. Examples of alkenyl include, but are not limited to, ethenyl (vinyl), propenyl, allyl, butenyl, methylbutenyl, butadienyl, pentenyl, pentadienyl, hexenyl, and hexadienyl.

The term "alkynyl" as used in the present specification refers to a monovalent group resulting from the loss of one hydrogen atom from an aliphatic unsaturated hydrocarbon having at least one triple bond. For example, "alkynyl" has 2 to 10, 2 to 8, 2 to 6, 2 to 5, 2 to 4, 2 to 3, 3 to 6, 4 to 6, 4 to 7, or 4 to 8 carbon atoms. The alkynyl group may be linear or branched. Examples of "alkynyl" include, but are not limited to, ethynyl, propargyl, butynyl, methylbutynyl, pentynyl, and hexynyl.

The term "hydroxyalkyl" as used in the present specification refers to an alkyl having hydroxy. Examples of "hydroxyalkyl" include, but are not limited to, hydroxymethyl, hydroxyethyl, 2-hydroxy-2-propyl, and hydroxypentyl.

The term "aminoalkyl" as used in the present specification refers to an alkyl having amino. Examples of "aminoalkyl" include, but are not limited to, aminomethyl and aminoethyl.

The term "halogen" or "halo" as used in the present specification refers to fluoro (-F), chloro (-CI), bromo (-Br), and iodo (-I).

The term "hydroxy" as used in the present specification refers to -OH.

The term "amino" as used in the present specification refers to -NH₂.

The term "salt" as used in the present specification has the broadest meaning as used in the technical field of the present disclosure, and generally refers to a salt that can be formed using an acid or a base, including an inorganic acid or inorganic base, or an organic acid or organic base. Examples of salts include, but are not limited to, alkali metal salts such as, but are not limited to, lithium, potassium, and sodium; alkaline earth metal salts such as, but are not limited to, barium, calcium, and magnesium; transition metal salts such as, but are not limited to, zinc; and other metal salts such as, but are not limited to, disodium hydrogen phosphate and disodium phosphate; mineral acid salts such as, but are not limited to, hydrochlorides and sulfates; organic acid salts such as, but are not limited to, acetates, lactates, maleates, malates, tartrates, citrates, ascorbates, succinates, butyrates, valerates, fumarates, and organic sulfonates; and amine salts such as, but are not limited to, lysine, N,N'-dibenzylethylenediamine, chloroprocaine, choline, ammonia, diethanolamine and other hydroxyalkylamines, ethylenediamine, N-methylglucamine, procaine, N-benzylphenethylamine, 1-p-chlorobenzyl-2-pyrrolidin-1'-ylmethyl benzimidazole, diethylamine and other alkylamines, piperazine, and tris(hydroxymethyl)aminomethane. The salts may also include acid addition salts such as carboxylic acid addition salts or dicarboxylic acid addition salts (e.g., maleate or fumarate addition salts), and base addition salts.

The term "pharmaceutically acceptable" as used in the present specification means that it is not harmful to a subject, such as a mammal including a human, particularly a human.

The term "solvate" as used in the present specification has the broadest meaning as used in the technical field of the present disclosure, and generally refers to a solvent-containing compound formed by the association of one or more solvent molecules with a compound. Examples of the solvent include, but are not limited to, water, methanol, ethanol, n-propanol, isopropanol, and acetic acid. The solvates include, for example, monosolvates, disolvates, trisolvates, and tetrasolvates. When the solvent is water, the solvate is a hydrate.

The term "stereoisomer" as used in the present specification has the broadest meaning as used in the technical field of the present disclosure. The stereoisomer includes diastereomers (e.g., cis-trans isomers) and enantiomers.

The term "tautomer" as used in the present specification has the broadest meaning as used in the technical field of the present disclosure. The tautomer includes keto-enol tautomer.

The term "deuterium-labeled compound" as used in the present specification refers to a compound in which at least one hydrogen atom in the compound structure is deuterium.

The term "subject" as used in the present specification refers to an animal, typically mammals including humans and non-human animals. Non-human animals include, for example, non-human mammals such as rats, mice, guinea pigs, rabbits, monkeys, dogs, cats, or miniature pigs. When the subject is a human, the subject may also be referred to as a "test subject" or a "patient." When the subject is a human, the subject may be either male or female. When the subject is a human, the age of the subject is not particularly limited, and may be, for example, a newborn, an infant, a toddler, a child (juvenile), a young adult, an adult, a middle-aged person, or an elderly person.

The term "about" as used in the present specification has the broadest meaning as used in the technical field of the present disclosure, and generally refers to an acceptable error range of the indicated numerical value. For example, the term "about" refers to a variation of ±0.1-20%, ±0.1-10%, ±0.1-5%, ±0.1-1.0%, or ±0.1-0.5% from a given value or value range.

The term "treatment" as used in the present specification has the broadest meaning as used in the technical field of the present disclosure, and generally refers to alleviating, reducing, improving, or eliminating symptoms associated with a particular disease or disorder.

The term "prevention" as used in the present specification has the broadest meaning as used in the technical field of the present disclosure, and generally refers to preventing the occurrence of symptoms associated with a particular disease or disorder in advance, such as administering a drug to a patient at risk of a psychiatric disorder before the onset of the symptoms.

The term "management" as used in the context of a particular disease or disorder in the present specification has the broadest meaning as used in the technical field of the present disclosure, and includes preventing recurrence of the particular disease or disorder, prolonging the duration of remission of a patient who has suffered from the particular disease or disorder, and/or maintaining a reduction or avoidance of the severity of symptoms associated with the particular disease or disorder.

The terms "carrier" and "excipient" as used in the present specification have the broadest meanings as used in the technical field of the present disclosure, and generally refer to non-toxic and inert solid, semi-solid, or liquid substances that are commonly used according to the dosage form or the like.

The term "effective amount" or "amount effective for ..." as used in the present specification refers to an amount of a compound or composition that can produce a desired effect. In some embodiments, a "therapeutically effective amount" refers to an amount of a compound or pharmaceutical composition that enables treatment, prevention, or management of a disease or disorder.

In the present specification, in contexts particularly related to concentrations or the like, the terms "high," "increased," and "excessive" refer to, for example, levels that are higher or increased compared to those in a healthy subject, higher or increased compared to the subject's own past levels, statistically significantly higher or increased, or greater than the median of a normal control group. The term "high," "increased," or "greater than the median" means, for example, values that are about 1.2-fold, about 1.5-fold, about 2-fold, about 3-fold, about 4-fold, about 5-fold, about 10-fold, about 15-fold, about 20-fold, about 30-fold, about 50-fold, about 100-fold or more higher, increased, or greater than the median. The terms "unaffected," "no change," and "no difference" may mean, for example, that the value is the same as that in healthy subjects, the same as that in subjects without UGT1A1 gene mutations, the same as the subject's own past value, not statistically significantly different, or equal to the median of a normal control group. The terms "the same" and "no significant difference" may mean, for example, a p-value of 0.05 or less or 0.01 or less, or a difference of about 0%, about 0.1%, about 0.2%, about 0.3%, about 0.4%, about 0.5%, about 1%, about 2%, about 3%, about 4%, about 5%, about 10%, about 20%, or about 30%.

### (2. Disorder and Subject)

The disorders to be treated, prevented, or managed by the present disclosure include BIND, psychiatric disorders associated with jaundice, or psychiatric disorders caused by UGT1A1 gene mutations. In one embodiment, the disorder to be treated, prevented, or managed by the present disclosure is one or more (e.g., one, two, or three) disorders selected from the group consisting of BIND, psychiatric disorders associated with jaundice, and psychiatric disorders caused by UGT1A1 gene mutations. In one embodiment, the disorder to be treated, prevented, or managed by the present disclosure is BIND. In one embodiment, the disorder to be treated, prevented, or managed by the present disclosure is a psychiatric disorder associated with jaundice. In one embodiment, the disorder to be treated, prevented, or managed by the present disclosure is a psychiatric disorder caused by UGT1A1 gene mutations.

The term "psychiatric disorders associated with jaundice" includes the co-occurrence of jaundice and a psychiatric disorder. In this case, jaundice may develop first followed by a psychiatric disorder, a psychiatric disorder may develop first followed by jaundice, or both jaundice and the psychiatric disorder may occur simultaneously. In certain embodiments, BIND or a psychiatric disorder caused by UGT1A1 gene mutations is accompanied by jaundice. In some specific embodiments, the jaundice is constitutional hyperbilirubinemia.

The term "psychiatric disorder caused by UGT1A1 gene mutations" refers to a psychiatric disorder that occurs as a result of a UGT1A1 gene mutation.

Specific examples of disorders to be treated, prevented, or managed by the present disclosure include, but are not limited to, UGT1A1 gene mutation-inducing delirium, dementia, amnestic disorders, other cognitive disorders, mental dysfunctions due to general medical conditions, substance-related disorders, schizophrenia and other psychotic disorders, mood disorders, anxiety disorders, somatoform disorders, factitious disorders, dissociative disorders, sexual disorders and gender identity disorders, eating disorders, sleep disorders, impulse-control disorders not elsewhere classified, adjustment disorders, personality disorders or attention deficit hyperactivity disorder (ADHD), and the like. Preferably, the psychiatric disorder to be treated, prevented, or managed by the present disclosure is schizophrenia caused by UGT1A1 gene mutations or attention deficit hyperactivity disorder (ADHD) caused by UGT1A1 gene mutations.

Other specific examples of disorders to be treated, prevented, or managed by the present disclosure include, but are not limited to, jaundice-accompanying delirium, dementia, amnestic disorders, other cognitive disorders, mental dysfunctions due to general medical conditions, substance-related disorders, schizophrenia and other psychotic disorders, mood disorders, anxiety disorders, somatoform disorders, factitious disorders, dissociative disorders, sexual disorders and gender identity disorders, eating disorders, sleep disorders, impulse-control disorders not elsewhere classified, adjustment disorders, personality disorders or attention deficit hyperactivity disorder (ADHD). Preferably, the psychiatric disorder to be treated, prevented, or managed by the present disclosure is schizophrenia associated with jaundice or attention deficit hyperactivity disorder (ADHD) associated with jaundice.

UGT1A1 gene mutation is known to those skilled in the art (see, for example, Non-Patent Literature 8). The term "UGT1A1 gene mutation" may refer to, for example, a mutation in one or more bases or a part of the bases of UGT1A1 gene, resulting in a decrease (e.g., a decrease of about 5%, about 10%, about 20%, about 30%, about 40%, about 50%, about 60%, about 70%, about 80%, about 90%, or about 95% or more) or loss of activity of the UGT1A1 gene compared to normal. Examples of UGT1A1 gene mutations include mutations in the promoter region, mutations in the coding region, frameshift mutations (specifically, frameshift mutations caused by deletion of one base (G)), homozygous Y486D, homozygous insertion of TA into the TATA box in the promoter region (TA7/7), homozygous G71R, a combination of TA7/6 and heterozygous G71R, homozygous or heterozygous Y486D and P229Q, heterozygous P364L, homozygous or heterozygous TA7, G71R, and heterozygous Y486D, and any combination of one or more of these.

The BIND or psychiatric disorder to be treated, prevented, or managed by the present disclosure may be caused by excessive serotonin transmission in the brain. UGT1A1 gene mutation may cause excessive serotonin transmission in the brain without affecting dopamine neurons, transmission, and/or concentration in the brain. Accordingly, in certain embodiments, the psychiatric disorder to be treated, prevented, or managed by the present disclosure is accompanied by excessive serotonin transmission in the brain but cause no change in dopamine transmission in the brain. UGT1A1 gene mutation may increase serotonin concentration in the brain and/or the number of serotonin neurons in the brain. Accordingly, in certain embodiments, the psychiatric disorder to be treated, prevented, or managed by the present disclosure is caused by increased serotonin concentration in the brain and/or increased number of serotonin neurons in the brain. UGT1A1 gene mutation may increase bilirubin level in the blood or urine and, as a result, cause jaundice. Accordingly, in certain embodiments, the psychiatric disorder to be treated, prevented, or managed by the present disclosure is associated with increased bilirubin level in the blood or urine. In certain embodiments, the psychiatric disorder to be treated, prevented, or managed by the present disclosure is associated with jaundice.

In one embodiment, the subject to be treated, prevented, or managed by the present disclosure has BIND, a psychiatric disorder associated with jaundice, or a psychiatric disorder caused by UGT1A1 gene mutations. In one embodiment, the subject to be treated, prevented, or managed by the present disclosure has one or more (e.g., one, two, or three) disorders selected from the group consisting of BIND, psychiatric disorders associated with jaundice, and psychiatric disorders caused by UGT1A1 gene mutations.

In some embodiments, the subject to be treated, prevented, or managed by the present disclosure has BIND. In some embodiments, the subject to be treated, prevented, or managed by the present disclosure has a UGT1A1 gene mutation. In some embodiments, the subject to be treated, prevented, or managed by the present disclosure has jaundice. In some embodiments, the subject to be treated, prevented, or managed by the present disclosure has, in the biological sample, a high bilirubin level, for example, a bilirubin level higher than that of a healthy subject.

In some embodiments, the subject to be treated, prevented, or managed by the present disclosure has BIND and jaundice. In some embodiments, the subject to be treated, prevented, or managed by the present disclosure has BIND and a UGT1A1 gene mutation. In some embodiments, the subject to be treated, prevented, or managed by the present disclosure has BIND, UGT1A1 gene mutation, and jaundice. In some embodiments, the subject to be treated, prevented, or managed by the present disclosure has a UGT1A1 gene mutation, and jaundice.

In some specific embodiments, the subject to be treated, prevented, or managed by the present disclosure has BIND and has, in the biological sample, a high bilirubin level compared with that of a healthy subject, or jaundice.

In some embodiments, the subject to be treated, prevented, or managed by the present disclosure has a psychiatric disorder. In some embodiments, the subject to be treated, prevented, or managed by the present disclosure has a psychiatric disorder and UGT1A1 gene mutation. In some embodiments, the subject to be treated, prevented, or managed by the present disclosure has a psychiatric disorder and jaundice. In some embodiments, the subject to be treated, prevented, or managed by the present disclosure has a psychiatric disorder, UGT1A1 gene mutation, and jaundice.

In some specific embodiments, the subject to be treated, prevented, or managed by the present disclosure has a psychiatric disorder and has, in the biological sample, a high bilirubin level compared with that of a healthy subject, or jaundice.

In some embodiments, the subject to be treated, prevented, or managed by the present disclosure has one or more (e.g., one, two, three, four, or five) conditions selected from the group consisting of a psychiatric disorder, BIND, jaundice, UGT1A1 gene mutation, and a high bilirubin level in the biological sample, for example, a bilirubin level higher than that of a healthy subject. The psychiatric disorder may include a psychiatric disorder associated with jaundice and/or a psychiatric disorder caused by UGT1A1 gene mutations.

In some specific embodiments, the jaundice is constitutional hyperbilirubinemia.

In some specific embodiments, the biological sample is blood or urine.

Whether the subject has BIND can be determined by methods known to those skilled in the art. For example, the presence or absence of BIND in a subject can be determined by examining the bilirubin level (total bilirubin level, direct bilirubin level, or indirect bilirubin level) in a biological sample derived from the subject, a UGT1A1 gene mutation, or a combination of two or more, three or more, or four of these factors. For example, if the blood bilirubin level is increased in the subject, such as when the blood bilirubin level in the subject is higher than that of a healthy subject, it can be determined that the subject has BIND.

Whether the subject has jaundice can be determined by methods known to those skilled in the art. For example, constitutional hyperbilirubinemia can be tested by a blood test, in which the indirect bilirubin level in the blood is measured. For example, if the blood indirect bilirubin level is increased in the subject, such as when the blood bilirubin level in the subject is higher than that of a healthy subject, it can be determined that the subject has jaundice.

The total bilirubin level and direct bilirubin level can be measured using, for example, total bilirubin blood test kit CicaLiquid D-BIL (KANTO KAGAKU), a method using vanadic acid (see Japanese Journal of Clinical Chemistry 22: 116-122, 1993, this publication is incorporated herein by reference), direct bilirubin kit Aqua-auto Kainos D-BIL reagent (KAINOS Laboratories, Inc.), or total bilirubin measurement "serotec" T-BIL (serotec Co,.Ltd). Indirect bilirubin level generally cannot be measured directly, and is typically calculated by subtracting the direct bilirubin level from the total bilirubin level.

Whether the subject has a psychiatric disorder can be determined by methods known to those skilled in the art. For example, the determination can be made using diagnostic tests for various psychiatric disorders, interviews, electroencephalography, and CT scans, or a combination of one or more of these methods.

Whether the subject has a UGT1A1 gene mutation can be determined by methods known to those skilled in the art. For example, the Invader (registered trademark) UGT1A1 assay (Sekisui Medical Co., Ltd.) can be used as a reagent for determining UGT1A1 gene polymorphism.

UGT1A1 gene mutation may cause excessive serotonin transmission in the brain. Accordingly, in certain embodiments, the subject to be treated, prevented, or managed by the present disclosure is accompanied by excessive serotonin transmission in the brain. UGT1A1 gene mutation may cause excessive serotonin transmission in the brain without affecting dopamine neurons, transmission, and/or concentration in the brain. Therefore, in certain embodiments, the subject to be treated, prevented, or managed by the present disclosure exhibits no changes in dopamine neurons, transmission, and/or concentration in the brain. In certain embodiments, the subject to be treated, prevented, or managed by the present disclosure has excessive serotonin transmission in the brain but exhibits no changes in dopamine transmission in the brain. UGT1A1 gene mutation may increase serotonin concentration in the brain and/or the number of serotonin neurons in the brain. Accordingly, in certain embodiments, the subject to be treated, prevented, or managed by the present disclosure exhibits increased serotonin concentration in the brain and/or increased number of serotonin neurons in the brain. UGT1A1 gene mutation may increase bilirubin level in the blood or urine and, as a result, UGT1A1 gene mutation may cause jaundice. Accordingly, in certain embodiments, the subject to be treated, prevented, or managed by the present disclosure exhibits increased bilirubin level in the blood or urine. In certain embodiments, the subject to be treated, prevented, or managed by the present disclosure exhibits increased bilirubin levels in the blood or urine compared with that of a healthy subject.

The brain is, for example, one or more (e.g., one, two, three, or four) regions selected from the group consisting of the brainstem, frontal lobe, striatum, hippocampus, and nucleus originis (particularly raphe nuclei, e.g., dorsal raphe nucleus). Regarding serotonin, the brain is preferably the frontal lobe and hippocampus. Regarding dopamine, the brain is preferably the frontal lobe, striatum, and hippocampus.

### (3. 5-HT_{1A/1B} Receptor Agonist)

The 5-HT_{1A/1B} receptor agonist can be used to treat, prevent, or manage the disorders described in the present specification. The 5-HT_{1A/1B} receptor agonist can be used in the manufacture of a medicament for treating, preventing, or managing the disorders described in the present specification. The 5-HT_{1A/1B} receptor agonist can be used to treat, prevent, or manage the subjects described in the present specification. The 5-HT_{1A/1B} receptor agonist can be used in the manufacture of a medicament for treating, preventing, or managing the subjects described in the present specification.

The 5-HT_{1A/1B} receptor agonist can be used in the treatment, prevention, or management methods described in the present specification.

In one embodiment, the 5-HT_{1A/1B} receptor agonist is used to treat, prevent, or manage BIND, a psychiatric disorder associated with jaundice, or a psychiatric disorder caused by UGT1A1 gene mutation in a subject. In one embodiment, the 5-HT_{1A/1B} receptor agonist is used in the manufacture of a medicament for treating, preventing, or managing BIND, a psychiatric disorder associated with jaundice, or a psychiatric disorder caused by UGT1A1 gene mutation in a subject.

In one embodiment, the 5-HT_{1A/1B} receptor agonist is used to treat one or more (e.g., one, two, or three) disorders selected from the group consisting of BIND, a psychiatric disorder associated with jaundice, and a psychiatric disorder caused by UGT1A1 gene mutation in a subject. In one embodiment, the 5-HT_{1A/1B} receptor agonist is used to prevent one or more (e.g., one, two, or three) disorders selected from the group consisting of BIND, a psychiatric disorder associated with jaundice, and a psychiatric disorder caused by UGT1A1 gene mutation in a subject. In one embodiment, the 5-HT_{1A/1B} receptor agonist is used to manage one or more (e.g., one, two, or three) disorders selected from the group consisting of BIND, a psychiatric disorder associated with jaundice, and a psychiatric disorder caused by UGT1A1 gene mutation in a subject.

In one embodiment, the 5-HT_{1A/1B} receptor agonist is used to treat, prevent, or manage BIND in a subject. In one embodiment, the 5-HT_{1A/1B} receptor agonist is used to treat, prevent, or manage a psychiatric disorder associated with jaundice in a subject. In one embodiment, the 5-HT_{1A/1B} receptor agonist is used to treat, prevent, or manage a psychiatric disorder caused by UGT1A1 gene mutation in a subject.

In some embodiments, the 5-HT_{1A/1B} receptor agonist is used for treatment, prevention, or management methods for a psychiatric disorder in a subject.

The 5-HT_{1A/1B} receptor agonist can be used in the treatment, prevention, or management methods for a subject. In some embodiments, the subject has a psychiatric disorder. In some embodiments, the subject has BIND. In some embodiments, the subject has jaundice. In some embodiments, the subject has UGT1A1 gene mutation. In some embodiments, the subject has a high bilirubin level in the biological sample. In some embodiments, the subject has a high bilirubin level in the biological sample compared to that of a healthy subject. In some embodiments, the subject has one or more (e.g., one, two, three, four, or five) conditions selected from the group consisting of a psychiatric disorder, BIND, jaundice, UGT1A1 gene mutation, and a high bilirubin level in the biological sample, for example, a bilirubin level higher than that of a healthy subject.

In one embodiment, the 5-HT_{1A/1B} receptor agonist is a compound of the following formula (I), or a pharmaceutically acceptable salt, solvate, hydrate, stereoisomer, tautomer, deuterium-labeled compound, crystal, polymorph, or prodrug thereof: 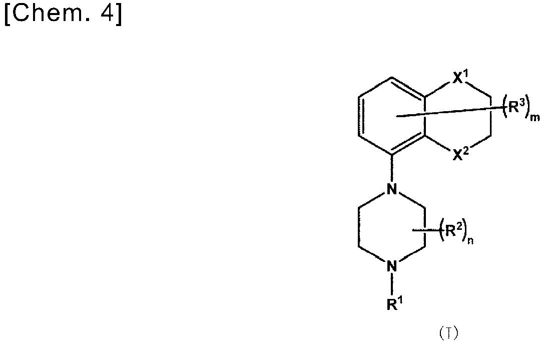
wherein R¹ is hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
R² and R³ are each independently, at each occurrence, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
X¹ and X² are each independently oxygen, sulfur, or -CR^{a}R^{b};
R^{a} and R^{b} are each independently hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
n is an integer of 0 to 4; and
m is an integer of 0 to 5.

In one embodiment, R¹ is hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl, and is preferably hydrogen. In one embodiment, R² and R³ are each independently, at each occurrence, halogen, hydroxy, amino, C₁₋₆ alkyl, C₂₋₆ alkenyl, C₂₋₆ alkynyl, C₁₋₆ hydroxyalkyl, or C₁₋₆ aminoalkyl. R² may be bonded at a bondable position on the piperazine ring. In one embodiment, R² may be bonded at a bondable position on the piperazine ring other than on the nitrogen atom. R³ may be bonded at a bondable position on the condensed ring (a condensed ring formed by the ring containing X¹ and X² and a benzene ring) other than X¹ and X². In one embodiment, X¹ and X² are each independently oxygen, sulfur, or -CR^{a}R^{b}, preferably oxygen. More preferably, both X¹ and X² are oxygen. In one embodiment, R^{a} and R^{b} are each independently hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl. In one embodiment, n is an integer of 0, 1, 2, 3 or 4, and is preferably 0. In one embodiment, m is an integer of 0, 1, 2, 3, 4 or 5, and is preferably 0.

In a specific embodiment, the compound of Formula (I) is eltoprazine (5-(piperazin-1-yl)-2,3-dihydro-1,4-benzodioxine). In a specific embodiment, the 5-HT_{1A/1B} receptor agonist is eltoprazine (5-(piperazin-1-yl)-2,3-dihydro-1,4-benzodioxine), or a pharmaceutically acceptable salt, solvate, hydrate, stereoisomer, tautomer, deuterium-labeled compound, crystal, polymorph, or prodrug thereof. Eltoprazine has the following structure:

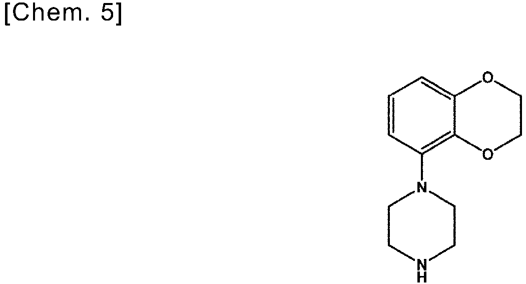

In a specific embodiment, the 5-HT_{1A/1B} receptor agonist or the compound of formula (I) is a compound selected from the group consisting of the following compounds, or a pharmaceutically acceptable salt, solvate, hydrate, stereoisomer, tautomer, deuterium-labeled compound, crystal, polymorph, or prodrug thereof:
5-(piperazin-1-yl)-2,3-dihydro-1,4-benzodioxine;
5-(2-chloropiperazin-1-yl)-2,3-dihydro-1,4-benzodioxine;
5-(3-chloropiperazin-1-yl)-2,3-dihydro-1,4-benzodioxine;
5-(4-chloropiperazin-1-yl)-2,3-dihydro-1,4-benzodioxine;
5-(2-fluoropiperazin-1-yl)-2,3-dihydro-1,4-benzodioxine;
5-(3-fluoropiperazin-1-yl)-2,3-dihydro-1,4-benzodioxine;
5-(4-fluoropiperazin-1-yl)-2,3-dihydro-1,4-benzodioxine;
5-(2-hydroxypiperazin-1-yl)-2,3-dihydro-1,4-benzodioxine;
5-(3-hydroxypiperazin-1-yl)-2,3-dihydro-1,4-benzodioxine;
5-(4-hydroxypiperazin-1-yl)-2,3-dihydro-1,4-benzodioxine;
5-(2-aminopiperazin-1-yl)-2,3-dihydro-1,4-benzodioxine;
5-(3-aminopiperazin-1-yl)-2,3-dihydro-1,4-benzodioxine;
5-(4-aminopiperazin-1-yl)-2,3-dihydro-1,4-benzodioxine;
5-(2-methylpiperazin-1-yl)-2,3-dihydro-1,4-benzodioxine;
5-(3-methylpiperazin-1-yl)-2,3-dihydro-1,4-benzodioxine;
5-(4-methylpiperazin-1-yl)-2,3-dihydro-1,4-benzodioxine;
5-(2-ethylpiperazin-1-yl)-2,3-dihydro-1,4-benzodioxine;
5-(3-ethylpiperazin-1-yl)-2,3-dihydro-1,4-benzodioxine;
5-(4-ethylpiperazin-1-yl)-2,3-dihydro-1,4-benzodioxine;
5-(2-(hydroxymethyl)piperazin-1-yl)-2,3-dihydro-1,4-benzodioxine;
5-(3-(hydroxymethyl)piperazin-1-yl)-2,3-dihydro-1,4-benzodioxine;
5-(4-(hydroxymethyl)piperazin-1-yl)-2,3-dihydro-1,4-benzodioxine;
5-(2-(1-hydroxyethyl)piperazin-1-yl)-2,3-dihydro-1,4-benzodioxine;
5-(3-(1-hydroxyethyl)piperazin-1-yl)-2,3-dihydro-1,4-benzodioxine;
5-(4-(1-hydroxyethyl)piperazin-1-yl)-2,3-dihydro-1,4-benzodioxine;
5-(2-(2-hydroxyethyl)piperazin-1-yl)-2,3-dihydro-1,4-benzodioxine;
5-(3-(2-hydroxyethyl)piperazin-1-yl)-2,3-dihydro-1,4-benzodioxine;
5-(4-(2-hydroxyethyl)piperazin-1-yl)-2,3-dihydro-1,4-benzodioxine;
5-(2-(aminomethyl)piperazin-1-yl)-2,3-dihydro-1,4-benzodioxine;
5-(3-(aminomethyl)piperazin-1-yl)-2,3-dihydro-1,4-benzodioxine;
5-(4-(aminomethyl)piperazin-1-yl)-2,3-dihydro-1,4-benzodioxine;
5-(2-(1-aminoethyl)piperazin-1-yl)-2,3-dihydro-1,4-benzodioxine;
5-(3-(1-aminoethyl)piperazin-1-yl)-2,3-dihydro-1,4-benzodioxine;
5-(4-(1-aminoethyl)piperazin-1-yl)-2,3-dihydro-1,4-benzodioxine;
5-(2-(2-aminoethyl)piperazin-1-yl)-2,3-dihydro-1,4-benzodioxine;
5-(3-(2-aminoethyl)piperazin-1-yl)-2,3-dihydro-1,4-benzodioxine;
5-(4-(2-aminoethyl)piperazin-1-yl)-2,3-dihydro-1,4-benzodioxine;
2-chloro-5-(piperazin-1-yl)-2,3-dihydro-1,4-benzodioxine;
3-chloro-5-(piperazin-1-yl)-2,3-dihydro-1,4-benzodioxine;
6-chloro-5-(piperazin-1-yl)-2,3-dihydro-1,4-benzodioxine;
7-chloro-5-(piperazin-1-yl)-2,3-dihydro-1,4-benzodioxine;
8-chloro-5-(piperazin-1-yl)-2,3-dihydro-1,4-benzodioxine;
2-fluoro-5-(piperazin-1-yl)-2,3-dihydro-1,4-benzodioxine;
3-fluoro-5-(piperazin-1-yl)-2,3-dihydro-1,4-benzodioxine;
6-fluoro-5-(piperazin-1-yl)-2,3-dihydro-1,4-benzodioxine;
7-fluoro-5-(piperazin-1-yl)-2,3-dihydro-1,4-benzodioxine;
8-fluoro-5-(piperazin-1-yl)-2,3-dihydro-1,4-benzodioxine;
2-hydroxy-5-(piperazin-1-yl)-2,3-dihydro-1,4-benzodioxine;
3-hydroxy-5-(piperazin-1-yl)-2,3-dihydro-1,4-benzodioxine;
6-hydroxy-5-(piperazin-1-yl)-2,3-dihydro-1,4-benzodioxine;
7-hydroxy-5-(piperazin-1-yl)-2,3-dihydro-1,4-benzodioxine;
8-hydroxy-5-(piperazin-1-yl)-2,3-dihydro-1,4-benzodioxine;
2-amino-5-(piperazin-1-yl)-2,3-dihydro-1,4-benzodioxine;
3-amino-5-(piperazin-1-yl)-2,3-dihydro-1,4-benzodioxine;
6-amino-5-(piperazin-1-yl)-2,3-dihydro-1,4-benzodioxine;
7-amino-5-(piperazin-1-yl)-2,3-dihydro-1,4-benzodioxine;
8-amino-5-(piperazin-1-yl)-2,3-dihydro-1,4-benzodioxine;
2-methyl-5-(piperazin-1-yl)-2,3-dihydro-1,4-benzodioxine;
3-methyl-5-(piperazin-1-yl)-2,3-dihydro-1,4-benzodioxine;
6-methyl-5-(piperazin-1-yl)-2,3-dihydro-1,4-benzodioxine;
7-methyl-5-(piperazin-1-yl)-2,3-dihydro-1,4-benzodioxine;
8-methyl-5-(piperazin-1-yl)-2,3-dihydro-1,4-benzodioxine;
2-ethyl-5-(piperazin-1-yl)-2,3-dihydro-1,4-benzodioxine;
3-ethyl-5-(piperazin-1-yl)-2,3-dihydro-1,4-benzodioxine;
6-ethyl-5-(piperazin-1-yl)-2,3-dihydro-1,4-benzodioxine;
7-ethyl-5-(piperazin-1-yl)-2,3-dihydro-1,4-benzodioxine;
8-ethyl-5-(piperazin-1-yl)-2,3-dihydro-1,4-benzodioxine;
2-(hydroxymethyl)-5-(piperazin-1-yl)-2,3-dihydro-1,4-benzodioxine;
3-(hydroxymethyl)-5-(piperazin-1-yl)-2,3-dihydro-1,4-benzodioxine;
6-(hydroxymethyl)-5-(piperazin-1-yl)-2,3-dihydro-1,4-benzodioxine;
7-(hydroxymethyl)-5-(piperazin-1-yl)-2,3-dihydro-1,4-benzodioxine;
8-(hydroxymethyl)-5-(piperazin-1-yl)-2,3-dihydro-1,4-benzodioxine;
2-(1-hydroxyethyl)-5-(piperazin-1-yl)-2,3-dihydro-1,4-benzodioxine;
3-(1-hydroxyethyl)-5-(piperazin-1-yl)-2,3-dihydro-1,4-benzodioxine;
6-(1-hydroxyethyl)-5-(piperazin-1-yl)-2,3-dihydro-1,4-benzodioxine;
7-(1-hydroxyethyl)-5-(piperazin-1-yl)-2,3-dihydro-1,4-benzodioxine;
8-(1-hydroxyethyl)-5-(piperazin-1-yl)-2,3-dihydro-1,4-benzodioxine;
2-(2-hydroxyethyl)-5-(piperazin-1-yl)-2,3-dihydro-1,4-benzodioxine;
3-(2-hydroxyethyl)-5-(piperazin-1-yl)-2,3-dihydro-1,4-benzodioxine;
6-(2-hydroxyethyl)-5-(piperazin-1-yl)-2,3-dihydro-1,4-benzodioxine;
7-(2-hydroxyethyl)-5-(piperazin-1-yl)-2,3-dihydro-1,4-benzodioxine;
8-(2-hydroxyethyl)-5-(piperazin-1-yl)-2,3-dihydro-1,4-benzodioxine;
2-(aminomethyl)-5-(piperazin-1-yl)-2,3-dihydro-1,4-benzodioxine;
3-(aminomethyl)-5-(piperazin-1-yl)-2,3-dihydro-1,4-benzodioxine;
6-(aminomethyl)-5-(piperazin-1-yl)-2,3-dihydro-1,4-benzodioxine;
7-(aminomethyl)-5-(piperazin-1-yl)-2,3-dihydro-1,4-benzodioxine;
8-(aminomethyl)-5-(piperazin-1-yl)-2,3-dihydro-1,4-benzodioxine;
2-(1-aminoethyl)-5-(piperazin-1-yl)-2,3-dihydro-1,4-benzodioxine;
3-(1-aminoethyl)-5-(piperazin-1-yl)-2,3-dihydro-1,4-benzodioxine;
6-(1-aminoethyl)-5-(piperazin-1-yl)-2,3-dihydro-1,4-benzodioxine;
7-(1-aminoethyl)-5-(piperazin-1-yl)-2,3-dihydro-1,4-benzodioxine;
8-(1-aminoethyl)-5-(piperazin-1-yl)-2,3-dihydro-1,4-benzodioxine;
2-(2-aminoethyl)-5-(piperazin-1-yl)-2,3-dihydro-1,4-benzodioxine;
3-(2-aminoethyl)-5-(piperazin-1-yl)-2,3-dihydro-1,4-benzodioxine;
6-(2-aminoethyl)-5-(piperazin-1-yl)-2,3-dihydro-1,4-benzodioxine;
7-(2-aminoethyl)-5-(piperazin-1-yl)-2,3-dihydro-1,4-benzodioxine;
8-(2-aminoethyl)-5-(piperazin-1-yl)-2,3-dihydro-1,4-benzodioxine;
5-(2,2-dichloropiperazin-1-yl)-2,3-dihydro-1,4-benzodioxine;
5-(2,3-dichloropiperazin-1-yl)-2,3-dihydro-1,4-benzodioxine;
5-(2,4-dichloropiperazin-1-yl)-2,3-dihydro-1,4-benzodioxine;
5-(2,5-dichloropiperazin-1-yl)-2,3-dihydro-1,4-benzodioxine;
5-(2,6-dichloropiperazin-1-yl)-2,3-dihydro-1,4-benzodioxine;
5-(3,3-dichloropiperazin-1-yl)-2,3-dihydro-1,4-benzodioxine;
5-(3,4-dichloropiperazin-1-yl)-2,3-dihydro-1,4-benzodioxine;
5-(3,5-dichloropiperazin-1-yl)-2,3-dihydro-1,4-benzodioxine;
5-(2,2-difluoropiperazin-1-yl)-2,3-dihydro-1,4-benzodioxine;
5-(2,3-difluoropiperazin-1-yl)-2,3-dihydro-1,4-benzodioxine;
5-(2,4-difluoropiperazin-1-yl)-2,3-dihydro-1,4-benzodioxine;
5-(2,5-difluoropiperazin-1-yl)-2,3-dihydro-1,4-benzodioxine;
5-(2,6-difluoropiperazin-1-yl)-2,3-dihydro-1,4-benzodioxine;
5-(3,3-difluoropiperazin-1-yl)-2,3-dihydro-1,4-benzodioxine;
5-(3,4-difluoropiperazin-1-yl)-2,3-dihydro-1,4-benzodioxine;
5-(3,5-difluoropiperazin-1-yl)-2,3-dihydro-1,4-benzodioxine;
5-(2,2-dihydroxypiperazin-1-yl)-2,3-dihydro-1,4-benzodioxine;
5-(2,3-dihydroxypiperazin-1-yl)-2,3-dihydro-1,4-benzodioxine;
5-(2,4-dihydroxypiperazin-1-yl)-2,3-dihydro-1,4-benzodioxine;
5-(2,5-dihydroxypiperazin-1-yl)-2,3-dihydro-1,4-benzodioxine;
5-(2,6-dihydroxypiperazin-1-yl)-2,3-dihydro-1,4-benzodioxine;
5-(3,3-dihydroxypiperazin-1-yl)-2,3-dihydro-1,4-benzodioxine;
5-(3,4-dihydroxypiperazin-1-yl)-2,3-dihydro-1,4-benzodioxine;
5-(3,5-dihydroxypiperazin-1-yl)-2,3-dihydro-1,4-benzodioxine;
5-(2,2-diaminopiperazin-1-yl)-2,3-dihydro-1,4-benzodioxine;
5-(2,3-diaminopiperazin-1-yl)-2,3-dihydro-1,4-benzodioxine;
5-(2,4-diaminopiperazin-1-yl)-2,3-dihydro-1,4-benzodioxine;
5-(2,5-diaminopiperazin-1-yl)-2,3-dihydro-1,4-benzodioxine;
5-(2,6-diaminopiperazin-1-yl)-2,3-dihydro-1,4-benzodioxine;
5-(3,3-diaminopiperazin-1-yl)-2,3-dihydro-1,4-benzodioxine;
5-(3,4-diaminopiperazin-1-yl)-2,3-dihydro-1,4-benzodioxine;
5-(3,5-diaminopiperazin-1-yl)-2,3-dihydro-1,4-benzodioxine;
5-(2,2-dimethylpiperazin-1-yl)-2,3-dihydro-1,4-benzodioxine;
5-(2,3-dimethylpiperazin-1-yl)-2,3-dihydro-1,4-benzodioxine;
5-(2,4-dimethylpiperazin-1-yl)-2,3-dihydro-1,4-benzodioxine;
5-(2,5-dimethylpiperazin-1-yl)-2,3-dihydro-1,4-benzodioxine;
5-(2,6-dimethylpiperazin-1-yl)-2,3-dihydro-1,4-benzodioxine;
5-(3,3-dimethylpiperazin-1-yl)-2,3-dihydro-1,4-benzodioxine;
5-(3,4-dimethylpiperazin-1-yl)-2,3-dihydro-1,4-benzodioxine;
5-(3,5-dimethylpiperazin-1-yl)-2,3-dihydro-1,4-benzodioxine;
5-(2,2-diethylpiperazin-1-yl)-2,3-dihydro-1,4-benzodioxine;
5-(2,3-diethylpiperazin-1-yl)-2,3-dihydro-1,4-benzodioxine;
5-(2,4-diethylpiperazin-1-yl)-2,3-dihydro-1,4-benzodioxine;
5-(2,5-diethylpiperazin-1-yl)-2,3-dihydro-1,4-benzodioxine;
5-(2,6-diethylpiperazin-1-yl)-2,3-dihydro-1,4-benzodioxine;
5-(3,3-diethylpiperazin-1-yl)-2,3-dihydro-1,4-benzodioxine;
5-(3,4-diethylpiperazin-1-yl)-2,3-dihydro-1,4-benzodioxine;
5-(3,5-diethylpiperazin-1-yl)-2,3-dihydro-1,4-benzodioxine;
5-(2,2-bis(hydroxymethyl)piperazin-1-yl)-2,3-dihydro-1,4-benzodioxine;
5-(2,3-bis(hydroxymethyl)piperazin-1-yl)-2,3-dihydro-1,4-benzodioxine;
5-(2,4-bis(hydroxymethyl)piperazin-1-yl)-2,3-dihydro-1,4-benzodioxine;
5-(2,5-bis(hydroxymethyl)piperazin-1-yl)-2,3-dihydro-1,4-benzodioxine;
5-(2,6-bis(hydroxymethyl)piperazin-1-yl)-2,3-dihydro-1,4-benzodioxine;
5-(3,3-bis(hydroxymethyl)piperazin-1-yl)-2,3-dihydro-1,4-benzodioxine;
5-(3,4-bis(hydroxymethyl)piperazin-1-yl)-2,3-dihydro-1,4-benzodioxine;
5-(3,5-bis(hydroxymethyl)piperazin-1-yl)-2,3-dihydro-1,4-benzodioxine;
5-(2,2-bis(aminomethyl)piperazin-1-yl)-2,3-dihydro-1,4-benzodioxine;
5-(2,3-bis(aminomethyl)piperazin-1-yl)-2,3-dihydro-1,4-benzodioxine;
5-(2,4-bis(aminomethyl)piperazin-1-yl)-2,3-dihydro-1,4-benzodioxine;
5-(2,5-bis(aminomethyl)piperazin-1-yl)-2,3-dihydro-1,4-benzodioxine;
5-(2,6-bis(aminomethyl)piperazin-1-yl)-2,3-dihydro-1,4-benzodioxine;
5-(3,3-bis(aminomethyl)piperazin-1-yl)-2,3-dihydro-1,4-benzodioxine;
5-(3,4-bis(aminomethyl)piperazin-1-yl)-2,3-dihydro-1,4-benzodioxine;
5-(3,5-bis(aminomethyl)piperazin-1-yl)-2,3-dihydro-1,4-benzodioxine;
2,2-dichloro-5-(piperazin-1-yl)-2,3-dihydro-1,4-benzodioxine;
2,3-dichloro-5-(piperazin-1-yl)-2,3-dihydro-1,4-benzodioxine;
2,6-dichloro-5-(piperazin-1-yl)-2,3-dihydro-1,4-benzodioxine;
2,7-dichloro-5-(piperazin-1-yl)-2,3-dihydro-1,4-benzodioxine;
2,8-dichloro-5-(piperazin-1-yl)-2,3-dihydro-1,4-benzodioxine;
3,3-dichloro-5-(piperazin-1-yl)-2,3-dihydro-1,4-benzodioxine;
3,6-dichloro-5-(piperazin-1-yl)-2,3-dihydro-1,4-benzodioxine;
3,7-dichloro-5-(piperazin-1-yl)-2,3-dihydro-1,4-benzodioxine;
3,8-dichloro-5-(piperazin-1-yl)-2,3-dihydro-1,4-benzodioxine;
6,7-dichloro-5-(piperazin-1-yl)-2,3-dihydro-1,4-benzodioxine;
6,8-dichloro-5-(piperazin-1-yl)-2,3-dihydro-1,4-benzodioxine;
7,8-dichloro-5-(piperazin-1-yl)-2,3-dihydro-1,4-benzodioxine;
2,2-difluoro-5-(piperazin-1-yl)-2,3-dihydro-1,4-benzodioxine;
2,3-difluoro-5-(piperazin-1-yl)-2,3-dihydro-1,4-benzodioxine;
2,6-difluoro-5-(piperazin-1-yl)-2,3-dihydro-1,4-benzodioxine;
2,7-difluoro-5-(piperazin-1-yl)-2,3-dihydro-1,4-benzodioxine;
2,8-difluoro-5-(piperazin-1-yl)-2,3-dihydro-1,4-benzodioxine;
3,3-difluoro-5-(piperazin-1-yl)-2,3-dihydro-1,4-benzodioxine;
3,6-difluoro-5-(piperazin-1-yl)-2,3-dihydro-1,4-benzodioxine;
3,7-difluoro-5-(piperazin-1-yl)-2,3-dihydro-1,4-benzodioxine;
3,8-difluoro-5-(piperazin-1-yl)-2,3-dihydro-1,4-benzodioxine;
6,7-difluoro-5-(piperazin-1-yl)-2,3-dihydro-1,4-benzodioxine;
6,8-difluoro-5-(piperazin-1-yl)-2,3-dihydro-1,4-benzodioxine;
7,8-difluoro-5-(piperazin-1-yl)-2,3-dihydro-1,4-benzodioxine;
2,2-dihydroxy-5-(piperazin-1-yl)-2,3-dihydro-1,4-benzodioxine;
2,3-dihydroxy-5-(piperazin-1-yl)-2,3-dihydro-1,4-benzodioxine;
2,6-dihydroxy-5-(piperazin-1-yl)-2,3-dihydro-1,4-benzodioxine;
2,7-dihydroxy-5-(piperazin-1-yl)-2,3-dihydro-1,4-benzodioxine;
2,8-dihydroxy-5-(piperazin-1-yl)-2,3-dihydro-1,4-benzodioxine;
3,3-dihydroxy-5-(piperazin-1-yl)-2,3-dihydro-1,4-benzodioxine;
3,6-dihydroxy-5-(piperazin-1-yl)-2,3-dihydro-1,4-benzodioxine;
3,7-dihydroxy-5-(piperazin-1-yl)-2,3-dihydro-1,4-benzodioxine;
3,8-dihydroxy-5-(piperazin-1-yl)-2,3-dihydro-1,4-benzodioxine;
6,7-dihydroxy-5-(piperazin-1-yl)-2,3-dihydro-1,4-benzodioxine;
6,8-dihydroxy-5-(piperazin-1-yl)-2,3-dihydro-1,4-benzodioxine;
7,8-dihydroxy-5-(piperazin-1-yl)-2,3-dihydro-1,4-benzodioxine;
2,2-diamino-5-(piperazin-1-yl)-2,3-dihydro-1,4-benzodioxine;
2,3-diamino-5-(piperazin-1-yl)-2,3-dihydro-1,4-benzodioxine;
2,6-diamino-5-(piperazin-1-yl)-2,3-dihydro-1,4-benzodioxine;
2,7-diamino-5-(piperazin-1-yl)-2,3-dihydro-1,4-benzodioxine;
2,8-diamino-5-(piperazin-1-yl)-2,3-dihydro-1,4-benzodioxine;
3,3-diamino-5-(piperazin-1-yl)-2,3-dihydro-1,4-benzodioxine;
3,6-diamino-5-(piperazin-1-yl)-2,3-dihydro-1,4-benzodioxine;
3,7-diamino-5-(piperazin-1-yl)-2,3-dihydro-1,4-benzodioxine;
3,8-diamino-5-(piperazin-1-yl)-2,3-dihydro-1,4-benzodioxine;
6,7-diamino-5-(piperazin-1-yl)-2,3-dihydro-1,4-benzodioxine;
6,8-diamino-5-(piperazin-1-yl)-2,3-dihydro-1,4-benzodioxine;
7,8-diamino-5-(piperazin-1-yl)-2,3-dihydro-1,4-benzodioxine;
2,2-dimethyl-5-(piperazin-1-yl)-2,3-dihydro-1,4-benzodioxine;
2,3-dimethyl-5-(piperazin-1-yl)-2,3-dihydro-1,4-benzodioxine;
2,6-dimethyl-5-(piperazin-1-yl)-2,3-dihydro-1,4-benzodioxine;
2,7-dimethyl-5-(piperazin-1-yl)-2,3-dihydro-1,4-benzodioxine;
2,8-dimethyl-5-(piperazin-1-yl)-2,3-dihydro-1,4-benzodioxine;
3,3-dimethyl-5-(piperazin-1-yl)-2,3-dihydro-1,4-benzodioxine;
3,6-dimethyl-5-(piperazin-1-yl)-2,3-dihydro-1,4-benzodioxine;
3,7-dimethyl-5-(piperazin-1-yl)-2,3-dihydro-1,4-benzodioxine;
3,8-dimethyl-5-(piperazin-1-yl)-2,3-dihydro-1,4-benzodioxine;
6,7-dimethyl-5-(piperazin-1-yl)-2,3-dihydro-1,4-benzodioxine;
6,8-dimethyl-5-(piperazin-1-yl)-2,3-dihydro-1,4-benzodioxine;
7,8-dimethyl-5-(piperazin-1-yl)-2,3-dihydro-1,4-benzodioxine;
2,2-diethyl-5-(piperazin-1-yl)-2,3-dihydro-1,4-benzodioxine;
2,3-diethyl-5-(piperazin-1-yl)-2,3-dihydro-1,4-benzodioxine;
2,6-diethyl-5-(piperazin-1-yl)-2,3-dihydro-1,4-benzodioxine;
2,7-diethyl-5-(piperazin-1-yl)-2,3-dihydro-1,4-benzodioxine;
2,8-diethyl-5-(piperazin-1-yl)-2,3-dihydro-1,4-benzodioxine;
3,3-diethyl-5-(piperazin-1-yl)-2,3-dihydro-1,4-benzodioxine;
3,6-diethyl-5-(piperazin-1-yl)-2,3-dihydro-1,4-benzodioxine;
3,7-diethyl-5-(piperazin-1-yl)-2,3-dihydro-1,4-benzodioxine;
3,8-diethyl-5-(piperazin-1-yl)-2,3-dihydro-1,4-benzodioxine;
6,7-diethyl-5-(piperazin-1-yl)-2,3-dihydro-1,4-benzodioxine;
6,8-diethyl-5-(piperazin-1-yl)-2,3-dihydro-1,4-benzodioxine;
7,8-diethyl-5-(piperazin-1-yl)-2,3-dihydro-1,4-benzodioxine;
2,2-bis(hydroxymethyl)-5-(piperazin-1-yl)-2,3-dihydro-1,4-benzodioxine;
2,3-bis(hydroxymethyl)-5-(piperazin-1-yl)-2,3-dihydro-1,4-benzodioxine;
2,6-bis(hydroxymethyl)-5-(piperazin-1-yl)-2,3-dihydro-1,4-benzodioxine;
2,7-bis(hydroxymethyl)-5-(piperazin-1-yl)-2,3-dihydro-1,4-benzodioxine;
2,8-bis(hydroxymethyl)-5-(piperazin-1-yl)-2,3-dihydro-1,4-benzodioxine;
3,3-bis(hydroxymethyl)-5-(piperazin-1-yl)-2,3-dihydro-1,4-benzodioxine;
3,6-bis(hydroxymethyl)-5-(piperazin-1-yl)-2,3-dihydro-1,4-benzodioxine;
3,7-bis(hydroxymethyl)-5-(piperazin-1-yl)-2,3-dihydro-1,4-benzodioxine;
3,8-bis(hydroxymethyl)-5-(piperazin-1-yl)-2,3-dihydro-1,4-benzodioxine;
6,7-bis(hydroxymethyl)-5-(piperazin-1-yl)-2,3-dihydro-1,4-benzodioxine;
6,8-bis(hydroxymethyl)-5-(piperazin-1-yl)-2,3-dihydro-1,4-benzodioxine;
7,8-bis(hydroxymethyl)-5-(piperazin-1-yl)-2,3-dihydro-1,4-benzodioxine;
2,2-bis(aminomethyl)-5-(piperazin-1-yl)-2,3-dihydro-1,4-benzodioxine;
2,3-bis(aminomethyl)-5-(piperazin-1-yl)-2,3-dihydro-1,4-benzodioxine;
2,6-bis(aminomethyl)-5-(piperazin-1-yl)-2,3-dihydro-1,4-benzodioxine;
2,7-bis(aminomethyl)-5-(piperazin-1-yl)-2,3-dihydro-1,4-benzodioxine;
2,8-bis(aminomethyl)-5-(piperazin-1-yl)-2,3-dihydro-1,4-benzodioxine;
3,3-bis(aminomethyl)-5-(piperazin-1-yl)-2,3-dihydro-1,4-benzodioxine;
3,6-bis(aminomethyl)-5-(piperazin-1-yl)-2,3-dihydro-1,4-benzodioxine;
3,7-bis(aminomethyl)-5-(piperazin-1-yl)-2,3-dihydro-1,4-benzodioxine;
3,8-bis(aminomethyl)-5-(piperazin-1-yl)-2,3-dihydro-1,4-benzodioxine;
6,7-bis(aminomethyl)-5-(piperazin-1-yl)-2,3-dihydro-1,4-benzodioxine;
6,8-bis(aminomethyl)-5-(piperazin-1-yl)-2,3-dihydro-1,4-benzodioxine;
7,8-bis(aminomethyl)-5-(piperazin-1-yl)-2,3-dihydro-1,4-benzodioxine;
2-chloro-5-(2-chloropiperazin-1-yl)-2,3-dihydro-1,4-benzodioxine;
3-chloro-5-(2-chloropiperazin-1-yl)-2,3-dihydro-1,4-benzodioxine;
6-chloro-5-(2-chloropiperazin-1-yl)-2,3-dihydro-1,4-benzodioxine;
7-chloro-5-(2-chloropiperazin-1-yl)-2,3-dihydro-1,4-benzodioxine;
8-chloro-5-(2-chloropiperazin-1-yl)-2,3-dihydro-1,4-benzodioxine;
2-chloro-5-(3-chloropiperazin-1-yl)-2,3-dihydro-1,4-benzodioxine;
3-chloro-5-(3-chloropiperazin-1-yl)-2,3-dihydro-1,4-benzodioxine;
6-chloro-5-(3-chloropiperazin-1-yl)-2,3-dihydro-1,4-benzodioxine;
7-chloro-5-(3-chloropiperazin-1-yl)-2,3-dihydro-1,4-benzodioxine;
8-chloro-5-(3-chloropiperazin-1-yl)-2,3-dihydro-1,4-benzodioxine;
2-chloro-5-(4-chloropiperazin-1-yl)-2,3-dihydro-1,4-benzodioxine;
3-chloro-5-(4-chloropiperazin-1-yl)-2,3-dihydro-1,4-benzodioxine;
6-chloro-5-(4-chloropiperazin-1-yl)-2,3-dihydro-1,4-benzodioxine;
7-chloro-5-(4-chloropiperazin-1-yl)-2,3-dihydro-1,4-benzodioxine;
8-chloro-5-(4-chloropiperazin-1-yl)-2,3-dihydro-1,4-benzodioxine;
2-fluoro-5-(2-fluoropiperazin-1-yl)-2,3-dihydro-1,4-benzodioxine;
3-fluoro-5-(2-fluoropiperazin-1-yl)-2,3-dihydro-1,4-benzodioxine;
6-fluoro-5-(2-fluoropiperazin-1-yl)-2,3-dihydro-1,4-benzodioxine;
7-fluoro-5-(2-fluoropiperazin-1-yl)-2,3-dihydro-1,4-benzodioxine;
8-fluoro-5-(2-fluoropiperazin-1-yl)-2,3-dihydro-1,4-benzodioxine;
2-fluoro-5-(3-fluoropiperazin-1-yl)-2,3-dihydro-1,4-benzodioxine;
3-fluoro-5-(3-fluoropiperazin-1-yl)-2,3-dihydro-1,4-benzodioxine;
6-fluoro-5-(3-fluoropiperazin-1-yl)-2,3-dihydro-1,4-benzodioxine;
7-fluoro-5-(3-fluoropiperazin-1-yl)-2,3-dihydro-1,4-benzodioxine;
8-fluoro-5-(3-fluoropiperazin-1-yl)-2,3-dihydro-1,4-benzodioxine;
2-fluoro-5-(4-fluoropiperazin-1-yl)-2,3-dihydro-1,4-benzodioxine;
3-fluoro-5-(4-fluoropiperazin-1-yl)-2,3-dihydro-1,4-benzodioxine;
6-fluoro-5-(4-fluoropiperazin-1-yl)-2,3-dihydro-1,4-benzodioxine;
7-fluoro-5-(4-fluoropiperazin-1-yl)-2,3-dihydro-1,4-benzodioxine;
8-fluoro-5-(4-fluoropiperazin-1-yl)-2,3-dihydro-1,4-benzodioxine;
2-hydroxy-5-(2-hydroxypiperazin-1-yl)-2,3-dihydro-1,4-benzodioxine;
3-hydroxy-5-(2-hydroxypiperazin-1-yl)-2,3-dihydro-1,4-benzodioxine;
6-hydroxy-5-(2-hydroxypiperazin-1-yl)-2,3-dihydro-1,4-benzodioxine;
7-hydroxy-5-(2-hydroxypiperazin-1-yl)-2,3-dihydro-1,4-benzodioxine;
8-hydroxy-5-(2-hydroxypiperazin-1-yl)-2,3-dihydro-1,4-benzodioxine;
2-hydroxy-5-(3-hydroxypiperazin-1-yl)-2,3-dihydro-1,4-benzodioxine;
3-hydroxy-5-(3-hydroxypiperazin-1-yl)-2,3-dihydro-1,4-benzodioxine;
6-hydroxy-5-(3-hydroxypiperazin-1-yl)-2,3-dihydro-1,4-benzodioxine;
7-hydroxy-5-(3-hydroxypiperazin-1-yl)-2,3-dihydro-1,4-benzodioxine;
8-hydroxy-5-(3-hydroxypiperazin-1-yl)-2,3-dihydro-1,4-benzodioxine;
2-hydroxy-5-(4-hydroxypiperazin-1-yl)-2,3-dihydro-1,4-benzodioxine;
3-hydroxy-5-(4-hydroxypiperazin-1-yl)-2,3-dihydro-1,4-benzodioxine;
6-hydroxy-5-(4-hydroxypiperazin-1-yl)-2,3-dihydro-1,4-benzodioxine;
7-hydroxy-5-(4-hydroxypiperazin-1-yl)-2,3-dihydro-1,4-benzodioxine;
8-hydroxy-5-(4-hydroxypiperazin-1-yl)-2,3-dihydro-1,4-benzodioxine;
2-amino-5-(2-aminopiperazin-1-yl)-2,3-dihydro-1,4-benzodioxine;
3-amino-5-(2-aminopiperazin-1-yl)-2,3-dihydro-1,4-benzodioxine;
6-amino-5-(2-aminopiperazin-1-yl)-2,3-dihydro-1,4-benzodioxine;
7-amino-5-(2-aminopiperazin-1-yl)-2,3-dihydro-1,4-benzodioxine;
8-amino-5-(2-aminopiperazin-1-yl)-2,3-dihydro-1,4-benzodioxine;
2-amino-5-(3-aminopiperazin-1-yl)-2,3-dihydro-1,4-benzodioxine;
3-amino-5-(3-aminopiperazin-1-yl)-2,3-dihydro-1,4-benzodioxine;
6-amino-5-(3-aminopiperazin-1-yl)-2,3-dihydro-1,4-benzodioxine;
7-amino-5-(3-aminopiperazin-1-yl)-2,3-dihydro-1,4-benzodioxine;
8-amino-5-(3-aminopiperazin-1-yl)-2,3-dihydro-1,4-benzodioxine;
2-amino-5-(4-aminopiperazin-1-yl)-2,3-dihydro-1,4-benzodioxine;
3-amino-5-(4-aminopiperazin-1-yl)-2,3-dihydro-1,4-benzodioxine;
6-amino-5-(4-aminopiperazin-1-yl)-2,3-dihydro-1,4-benzodioxine;
7-amino-5-(4-aminopiperazin-1-yl)-2,3-dihydro-1,4-benzodioxine;
8-amino-5-(4-aminopiperazin-1-yl)-2,3-dihydro-1,4-benzodioxine;
2-methyl-5-(2-methylpiperazin-1-yl)-2,3-dihydro-1,4-benzodioxine;
3-methyl-5-(2-methylpiperazin-1-yl)-2,3-dihydro-1,4-benzodioxine;
6-methyl-5-(2-methylpiperazin-1-yl)-2,3-dihydro-1,4-benzodioxine;
7-methyl-5-(2-methylpiperazin-1-yl)-2,3-dihydro-1,4-benzodioxine;
8-methyl-5-(2-methylpiperazin-1-yl)-2,3-dihydro-1,4-benzodioxine;
2-methyl-5-(3-methylpiperazin-1-yl)-2,3-dihydro-1,4-benzodioxine;
3-methyl-5-(3-methylpiperazin-1-yl)-2,3-dihydro-1,4-benzodioxine;
6-methyl-5-(3-methylpiperazin-1-yl)-2,3-dihydro-1,4-benzodioxine;
7-methyl-5-(3-methylpiperazin-1-yl)-2,3-dihydro-1,4-benzodioxine;
8-methyl-5-(3-methylpiperazin-1-yl)-2,3-dihydro-1,4-benzodioxine;
2-methyl-5-(4-methylpiperazin-1-yl)-2,3-dihydro-1,4-benzodioxine;
3-methyl-5-(4-methylpiperazin-1-yl)-2,3-dihydro-1,4-benzodioxine;
6-methyl-5-(4-methylpiperazin-1-yl)-2,3-dihydro-1,4-benzodioxine;
7-methyl-5-(4-methylpiperazin-1-yl)-2,3-dihydro-1,4-benzodioxine;
8-methyl-5-(4-methylpiperazin-1-yl)-2,3-dihydro-1,4-benzodioxine;
2-ethyl-5-(2-ethylpiperazin-1-yl)-2,3-dihydro-1,4-benzodioxine;
3-ethyl-5-(2-ethylpiperazin-1-yl)-2,3-dihydro-1,4-benzodioxine;
6-ethyl-5-(2-ethylpiperazin-1-yl)-2,3-dihydro-1,4-benzodioxine;
7-ethyl-5-(2-ethylpiperazin-1-yl)-2,3-dihydro-1,4-benzodioxine;
8-ethyl-5-(2-ethylpiperazin-1-yl)-2,3-dihydro-1,4-benzodioxine;
2-ethyl-5-(3-ethylpiperazin-1-yl)-2,3-dihydro-1,4-benzodioxine;
3-ethyl-5-(3-ethylpiperazin-1-yl)-2,3-dihydro-1,4-benzodioxine;
6-ethyl-5-(3-ethylpiperazin-1-yl)-2,3-dihydro-1,4-benzodioxine;
7-ethyl-5-(3-ethylpiperazin-1-yl)-2,3-dihydro-1,4-benzodioxine;
8-ethyl-5-(3-ethylpiperazin-1-yl)-2,3-dihydro-1,4-benzodioxine;
2-ethyl-5-(4-ethylpiperazin-1-yl)-2,3-dihydro-1,4-benzodioxine;
3-ethyl-5-(4-ethylpiperazin-1-yl)-2,3-dihydro-1,4-benzodioxine;
6-ethyl-5-(4-ethylpiperazin-1-yl)-2,3-dihydro-1,4-benzodioxine;
7-ethyl-5-(4-ethylpiperazin-1-yl)-2,3-dihydro-1,4-benzodioxine;
8-ethyl-5-(4-ethylpiperazin-1-yl)-2,3-dihydro-1,4-benzodioxine;
2-(hydroxymethyl)-5-(2-(hydroxymethyl)piperazin-1-yl)-2,3-dihydro-1,4-benzodioxine;
3-(hydroxymethyl)-5-(2-(hydroxymethyl)piperazin-1-yl)-2,3-dihydro-1,4-benzodioxine;
6-(hydroxymethyl)-5-(2-(hydroxymethyl)piperazin-1-yl)-2,3-dihydro-1,4-benzodioxine;
7-(hydroxymethyl)-5-(2-(hydroxymethyl)piperazin-1-yl)-2,3-dihydro-1,4-benzodioxine;
8-(hydroxymethyl)-5-(2-(hydroxymethyl)piperazin-1-yl)-2,3-dihydro-1,4-benzodioxine;
2-(hydroxymethyl)-5-(3-(hydroxymethyl)piperazin-1-yl)-2,3-dihydro-1,4-benzodioxine;
3-(hydroxymethyl)-5-(3-(hydroxymethyl)piperazin-1-yl)-2,3-dihydro-1,4-benzodioxine;
6-(hydroxymethyl)-5-(3-(hydroxymethyl)piperazin-1-yl)-2,3-dihydro-1,4-benzodioxine;
7-(hydroxymethyl)-5-(3-(hydroxymethyl)piperazin-1-yl)-2,3-dihydro-1,4-benzodioxine;
8-(hydroxymethyl)-5-(3-(hydroxymethyl)piperazin-1-yl)-2,3-dihydro-1,4-benzodioxine;
2-(hydroxymethyl)-5-(4-(hydroxymethyl)piperazin-1-yl)-2,3-dihydro-1,4-benzodioxine;
3-(hydroxymethyl)-5-(4-(hydroxymethyl)piperazin-1-yl)-2,3-dihydro-1,4-benzodioxine;
6-(hydroxymethyl)-5-(4-(hydroxymethyl)piperazin-1-yl)-2,3-dihydro-1,4-benzodioxine;
7-(hydroxymethyl)-5-(4-(hydroxymethyl)piperazin-1-yl)-2,3-dihydro-1,4-benzodioxine;
8-(hydroxymethyl)-5-(4-(hydroxymethyl)piperazin-1-yl)-2,3-dihydro-1,4-benzodioxine;
2-(aminomethyl)-5-(2-(aminomethyl)piperazin-1-yl)-2,3-dihydro-1,4-benzodioxine;
3-(aminomethyl)-5-(2-(aminomethyl)piperazin-1-yl)-2,3-dihydro-1,4-benzodioxine;
6-(aminomethyl)-5-(2-(aminomethyl)piperazin-1-yl)-2,3-dihydro-1,4-benzodioxine;
7-(aminomethyl)-5-(2-(aminomethyl)piperazin-1-yl)-2,3-dihydro-1,4-benzodioxine;
8-(aminomethyl)-5-(2-(aminomethyl)piperazin-1-yl)-2,3-dihydro-1,4-benzodioxine;
2-(aminomethyl)-5-(3-(aminomethyl)piperazin-1-yl)-2,3-dihydro-1,4-benzodioxine;
3-(aminomethyl)-5-(3-(aminomethyl)piperazin-1-yl)-2,3-dihydro-1,4-benzodioxine;
6-(aminomethyl)-5-(3-(aminomethyl)piperazin-1-yl)-2,3-dihydro-1,4-benzodioxine;
7-(aminomethyl)-5-(3-(aminomethyl)piperazin-1-yl)-2,3-dihydro-1,4-benzodioxine;
8-(aminomethyl)-5-(3-(aminomethyl)piperazin-1-yl)-2,3-dihydro-1,4-benzodioxine;
2-(aminomethyl)-5-(4-(aminomethyl)piperazin-1-yl)-2,3-dihydro-1,4-benzodioxine;
3-(aminomethyl)-5-(4-(aminomethyl)piperazin-1-yl)-2,3-dihydro-1,4-benzodioxine;
6-(aminomethyl)-5-(4-(aminomethyl)piperazin-1-yl)-2,3-dihydro-1,4-benzodioxine;
7-(aminomethyl)-5-(4-(aminomethyl)piperazin-1-yl)-2,3-dihydro-1,4-benzodioxine;
8-(aminomethyl)-5-(4-(aminomethyl)piperazin-1-yl)-2,3-dihydro-1,4-benzodioxine;
2-ethyl-5-(2-methylpiperazin-1-yl)-2,3-dihydro-1,4-benzodioxine; 3-ethyl-5-(2-methylpiperazin-1-yl)-2,3-dihydro-1,4-benzodioxine;
6-ethyl-5-(2-methylpiperazin-1-yl)-2,3-dihydro-1,4-benzodioxine;
7-ethyl-5-(2-methylpiperazin-1-yl)-2,3-dihydro-1,4-benzodioxine;
8-ethyl-5-(2-methylpiperazin-1-yl)-2,3-dihydro-1,4-benzodioxine;
2-ethyl-5-(3-methylpiperazin-1-yl)-2,3-dihydro-1,4-benzodioxine;
3-ethyl-5-(3-methylpiperazin-1-yl)-2,3-dihydro-1,4-benzodioxine;
6-ethyl-5-(3-methylpiperazin-1-yl)-2,3-dihydro-1,4-benzodioxine;
7-ethyl-5-(3-methylpiperazin-1-yl)-2,3-dihydro-1,4-benzodioxine;
8-ethyl-5-(3-methylpiperazin-1-yl)-2,3-dihydro-1,4-benzodioxine;
2-ethyl-5-(4-methylpiperazin-1-yl)-2,3-dihydro-1,4-benzodioxine;
3-ethyl-5-(4-methylpiperazin-1-yl)-2,3-dihydro-1,4-benzodioxine;
6-ethyl-5-(4-methylpiperazin-1-yl)-2,3-dihydro-1,4-benzodioxine;
7-ethyl-5-(4-methylpiperazin-1-yl)-2,3-dihydro-1,4-benzodioxine;
8-ethyl-5-(4-methylpiperazin-1-yl)-2,3-dihydro-1,4-benzodioxine;
5-(2-ethylpiperazin-1-yl)-2-methyl-2,3-dihydro-1,4-benzodioxine;
5-(2-ethylpiperazin-1-yl)-3-methyl-2,3-dihydro-1,4-benzodioxine;
5-(2-ethylpiperazin-1-yl)-6-methyl-2,3-dihydro-1,4-benzodioxine;
5-(2-ethylpiperazin-1-yl)-7-methyl-2,3-dihydro-1,4-benzodioxine;
5-(2-ethylpiperazin-1-yl)-8-methyl-2,3-dihydro-1,4-benzodioxine;
5-(3-ethylpiperazin-1-yl)-2-methyl-2,3-dihydro-1,4-benzodioxine;
5-(3-ethylpiperazin-1-yl)-3-methyl-2,3-dihydro-1,4-benzodioxine;
5-(3-ethylpiperazin-1-yl)-6-methyl-2,3-dihydro-1,4-benzodioxine;
5-(3-ethylpiperazin-1-yl)-7-methyl-2,3-dihydro-1,4-benzodioxine;
5-(3-ethylpiperazin-1-yl)-8-methyl-2,3-dihydro-1,4-benzodioxine;
5-(4-ethylpiperazin-1-yl)-2-methyl-2,3-dihydro-1,4-benzodioxine;
5-(4-ethylpiperazin-1-yl)-3-methyl-2,3-dihydro-1,4-benzodioxine;
5-(4-ethylpiperazin-1-yl)-6-methyl-2,3-dihydro-1,4-benzodioxine;
5-(4-ethylpiperazin-1-yl)-7-methyl-2,3-dihydro-1,4-benzodioxine;
5-(4-ethylpiperazin-1-yl)-8-methyl-2,3-dihydro-1,4-benzodioxine;
2-chloro-5-(2-methylpiperazin-1-yl)-2,3-dihydro-1,4-benzodioxine;
3-chloro-5-(2-methylpiperazin-1-yl)-2,3-dihydro-1,4-benzodioxine;
6-chloro-5-(2-methylpiperazin-1-yl)-2,3-dihydro-1,4-benzodioxine;
7-chloro-5-(2-methylpiperazin-1-yl)-2,3-dihydro-1,4-benzodioxine;
8-chloro-5-(2-methylpiperazin-1-yl)-2,3-dihydro-1,4-benzodioxine;
2-chloro-5-(3-methylpiperazin-1-yl)-2,3-dihydro-1,4-benzodioxine;
3-chloro-5-(3-methylpiperazin-1-yl)-2,3-dihydro-1,4-benzodioxine;
6-chloro-5-(3-methylpiperazin-1-yl)-2,3-dihydro-1,4-benzodioxine;
7-chloro-5-(3-methylpiperazin-1-yl)-2,3-dihydro-1,4-benzodioxine;
8-chloro-5-(3-methylpiperazin-1-yl)-2,3-dihydro-1,4-benzodioxine;
2-chloro-5-(4-methylpiperazin-1-yl)-2,3-dihydro-1,4-benzodioxine;
3-chloro-5-(4-methylpiperazin-1-yl)-2,3-dihydro-1,4-benzodioxine;
6-chloro-5-(4-methylpiperazin-1-yl)-2,3-dihydro-1,4-benzodioxine;
7-chloro-5-(4-methylpiperazin-1-yl)-2,3-dihydro-1,4-benzodioxine;
8-chloro-5-(4-methylpiperazin-1-yl)-2,3-dihydro-1,4-benzodioxine;
5-(2-chloropiperazin-1-yl)-2-methyl-2,3-dihydro-1,4-benzodioxine;
5-(2-chloropiperazin-1-yl)-3-methyl-2,3-dihydro-1,4-benzodioxine;
5-(2-chloropiperazin-1-yl)-6-methyl-2,3-dihydro-1,4-benzodioxine;
5-(2-chloropiperazin-1-yl)-7-methyl-2,3-dihydro-1,4-benzodioxine;
5-(2-chloropiperazin-1-yl)-8-methyl-2,3-dihydro-1,4-benzodioxine;
5-(3-chloropiperazin-1-yl)-2-methyl-2,3-dihydro-1,4-benzodioxine;
5-(3-chloropiperazin-1-yl)-3-methyl-2,3-dihydro-1,4-benzodioxine;
5-(3-chloropiperazin-1-yl)-6-methyl-2,3-dihydro-1,4-benzodioxine;
5-(3-chloropiperazin-1-yl)-7-methyl-2,3-dihydro-1,4-benzodioxine;
5-(3-chloropiperazin-1-yl)-8-methyl-2,3-dihydro-1,4-benzodioxine;
5-(4-chloropiperazin-1-yl)-2-methyl-2,3-dihydro-1,4-benzodioxine;
5-(4-chloropiperazin-1-yl)-3-methyl-2,3-dihydro-1,4-benzodioxine;
5-(4-chloropiperazin-1-yl)-6-methyl-2,3-dihydro-1,4-benzodioxine;
5-(4-chloropiperazin-1-yl)-7-methyl-2,3-dihydro-1,4-benzodioxine;
5-(4-chloropiperazin-1-yl)-8-methyl-2,3-dihydro-1,4-benzodioxine;
2-hydroxy-5-(2-methylpiperazin-1-yl)-2,3-dihydro-1,4-benzodioxine;
3-hydroxy-5-(2-methylpiperazin-1-yl)-2,3-dihydro-1,4-benzodioxine;
6-hydroxy-5-(2-methylpiperazin-1-yl)-2,3-dihydro-1,4-benzodioxine;
7-hydroxy-5-(2-methylpiperazin-1-yl)-2,3-dihydro-1,4-benzodioxine;
8-hydroxy-5-(2-methylpiperazin-1-yl)-2,3-dihydro-1,4-benzodioxine;
2-hydroxy-5-(3-methylpiperazin-1-yl)-2,3-dihydro-1,4-benzodioxine;
3-hydroxy-5-(3-methylpiperazin-1-yl)-2,3-dihydro-1,4-benzodioxine;
6-hydroxy-5-(3-methylpiperazin-1-yl)-2,3-dihydro-1,4-benzodioxine;
7-hydroxy-5-(3-methylpiperazin-1-yl)-2,3-dihydro-1,4-benzodioxine;
8-hydroxy-5-(3-methylpiperazin-1-yl)-2,3-dihydro-1,4-benzodioxine;
2-hydroxy-5-(4-methylpiperazin-1-yl)-2,3-dihydro-1,4-benzodioxine;
3-hydroxy-5-(4-methylpiperazin-1-yl)-2,3-dihydro-1,4-benzodioxine;
6-hydroxy-5-(4-methylpiperazin-1-yl)-2,3-dihydro-1,4-benzodioxine;
7-hydroxy-5-(4-methylpiperazin-1-yl)-2,3-dihydro-1,4-benzodioxine;
8-hydroxy-5-(4-methylpiperazin-1-yl)-2,3-dihydro-1,4-benzodioxine;
5-(2-hydroxypiperazin-1-yl)-2-methyl-2,3-dihydro-1,4-benzodioxine;
5-(2-hydroxypiperazin-1-yl)-3-methyl-2,3-dihydro-1,4-benzodioxine;
5-(2-hydroxypiperazin-1-yl)-6-methyl-2,3-dihydro-1,4-benzodioxine;
5-(2-hydroxypiperazin-1-yl)-7-methyl-2,3-dihydro-1,4-benzodioxine;
5-(2-hydroxypiperazin-1-yl)-8-methyl-2,3-dihydro-1,4-benzodioxine;
5-(3-hydroxypiperazin-1-yl)-2-methyl-2,3-dihydro-1,4-benzodioxine;
5-(3-hydroxypiperazin-1-yl)-3-methyl-2,3-dihydro-1,4-benzodioxine;
5-(3-hydroxypiperazin-1-yl)-6-methyl-2,3-dihydro-1,4-benzodioxine;
5-(3-hydroxypiperazin-1-yl)-7-methyl-2,3-dihydro-1,4-benzodioxine;
5-(3-hydroxypiperazin-1-yl)-8-methyl-2,3-dihydro-1,4-benzodioxine;
5-(4-hydroxypiperazin-1-yl)-2-methyl-2,3-dihydro-1,4-benzodioxine;
5-(4-hydroxypiperazin-1-yl)-3-methyl-2,3-dihydro-1,4-benzodioxine;
5-(4-hydroxypiperazin-1-yl)-6-methyl-2,3-dihydro-1,4-benzodioxine;
5-(4-hydroxypiperazin-1-yl)-7-methyl-2,3-dihydro-1,4-benzodioxine;
5-(4-hydroxypiperazin-1-yl)-8-methyl-2,3-dihydro-1,4-benzodioxine;
2-amino-5-(2-methylpiperazin-1-yl)-2,3-dihydro-1,4-benzodioxine;
3-amino-5-(2-methylpiperazin-1-yl)-2,3-dihydro-1,4-benzodioxine;
6-amino-5-(2-methylpiperazin-1-yl)-2,3-dihydro-1,4-benzodioxine;
7-amino-5-(2-methylpiperazin-1-yl)-2,3-dihydro-1,4-benzodioxine;
8-amino-5-(2-methylpiperazin-1-yl)-2,3-dihydro-1,4-benzodioxine;
2-amino-5-(3-methylpiperazin-1-yl)-2,3-dihydro-1,4-benzodioxine;
3-amino-5-(3-methylpiperazin-1-yl)-2,3-dihydro-1,4-benzodioxine;
6-amino-5-(3-methylpiperazin-1-yl)-2,3-dihydro-1,4-benzodioxine;
7-amino-5-(3-methylpiperazin-1-yl)-2,3-dihydro-1,4-benzodioxine;
8-amino-5-(3-methylpiperazin-1-yl)-2,3-dihydro-1,4-benzodioxine;
2-amino-5-(4-methylpiperazin-1-yl)-2,3-dihydro-1,4-benzodioxine;
3-amino-5-(4-methylpiperazin-1-yl)-2,3-dihydro-1,4-benzodioxine;
6-amino-5-(4-methylpiperazin-1-yl)-2,3-dihydro-1,4-benzodioxine;
7-amino-5-(4-methylpiperazin-1-yl)-2,3-dihydro-1,4-benzodioxine;
8-amino-5-(4-methylpiperazin-1-yl)-2,3-dihydro-1,4-benzodioxine;
5-(2-aminopiperazin-1-yl)-2-methyl-2,3-dihydro-1,4-benzodioxine;
5-(2-aminopiperazin-1-yl)-3-methyl-2,3-dihydro-1,4-benzodioxine;
5-(2-aminopiperazin-1-yl)-6-methyl-2,3-dihydro-1,4-benzodioxine;
5-(2-aminopiperazin-1-yl)-7-methyl-2,3-dihydro-1,4-benzodioxine;
5-(2-aminopiperazin-1-yl)-8-methyl-2,3-dihydro-1,4-benzodioxine;
5-(3-aminopiperazin-1-yl)-2-methyl-2,3-dihydro-1,4-benzodioxine;
5-(3-aminopiperazin-1-yl)-3-methyl-2,3-dihydro-1,4-benzodioxine;
5-(3-aminopiperazin-1-yl)-6-methyl-2,3-dihydro-1,4-benzodioxine;
5-(3-aminopiperazin-1-yl)-7-methyl-2,3-dihydro-1,4-benzodioxine;
5-(3-aminopiperazin-1-yl)-8-methyl-2,3-dihydro-1,4-benzodioxine;
5-(4-aminopiperazin-1-yl)-2-methyl-2,3-dihydro-1,4-benzodioxine;
5-(4-aminopiperazin-1-yl)-3-methyl-2,3-dihydro-1,4-benzodioxine;
5-(4-aminopiperazin-1-yl)-6-methyl-2,3-dihydro-1,4-benzodioxine;
5-(4-aminopiperazin-1-yl)-7-methyl-2,3-dihydro-1,4-benzodioxine;
5-(4-aminopiperazin-1-yl)-8-methyl-2,3-dihydro-1,4-benzodioxine;
5-(2-chloropiperazin-1-yl)-2-hydroxy-2,3-dihydro-1,4-benzodioxine;
5-(2-chloropiperazin-1-yl)-3-hydroxy-2,3-dihydro-1,4-benzodioxine;
5-(2-chloropiperazin-1-yl)-6-hydroxy-2,3-dihydro-1,4-benzodioxine;
5-(2-chloropiperazin-1-yl)-7-hydroxy-2,3-dihydro-1,4-benzodioxine;
5-(2-chloropiperazin-1-yl)-8-hydroxy-2,3-dihydro-1,4-benzodioxine;
5-(3-chloropiperazin-1-yl)-2-hydroxy-2,3-dihydro-1,4-benzodioxine;
5-(3-chloropiperazin-1-yl)-3-hydroxy-2,3-dihydro-1,4-benzodioxine;
5-(3-chloropiperazin-1-yl)-6-hydroxy-2,3-dihydro-1,4-benzodioxine;
5-(3-chloropiperazin-1-yl)-7-hydroxy-2,3-dihydro-1,4-benzodioxine;
5-(3-chloropiperazin-1-yl)-8-hydroxy-2,3-dihydro-1,4-benzodioxine;
5-(4-chloropiperazin-1-yl)-2-hydroxy-2,3-dihydro-1,4-benzodioxine;
5-(4-chloropiperazin-1-yl)-3-hydroxy-2,3-dihydro-1,4-benzodioxine;
5-(4-chloropiperazin-1-yl)-6-hydroxy-2,3-dihydro-1,4-benzodioxine;
5-(4-chloropiperazin-1-yl)-7-hydroxy-2,3-dihydro-1,4-benzodioxine;
5-(4-chloropiperazin-1-yl)-8-hydroxy-2,3-dihydro-1,4-benzodioxine;
2-chloro-5-(2-hydroxypiperazin-1-yl)-2,3-dihydro-1,4-benzodioxine;
3-chloro-5-(2-hydroxypiperazin-1-yl)-2,3-dihydro-1,4-benzodioxine;
6-chloro-5-(2-hydroxypiperazin-1-yl)-2,3-dihydro-1,4-benzodioxine;
7-chloro-5-(2-hydroxypiperazin-1-yl)-2,3-dihydro-1,4-benzodioxine;
8-chloro-5-(2-hydroxypiperazin-1-yl)-2,3-dihydro-1,4-benzodioxine;
2-chloro-5-(3-hydroxypiperazin-1-yl)-2,3-dihydro-1,4-benzodioxine;
3-chloro-5-(3-hydroxypiperazin-1-yl)-2,3-dihydro-1,4-benzodioxine;
6-chloro-5-(3-hydroxypiperazin-1-yl)-2,3-dihydro-1,4-benzodioxine;
7-chloro-5-(3-hydroxypiperazin-1-yl)-2,3-dihydro-1,4-benzodioxine;
8-chloro-5-(3-hydroxypiperazin-1-yl)-2,3-dihydro-1,4-benzodioxine;
2-chloro-5-(4-hydroxypiperazin-1-yl)-2,3-dihydro-1,4-benzodioxine;
3-chloro-5-(4-hydroxypiperazin-1-yl)-2,3-dihydro-1,4-benzodioxine;
6-chloro-5-(4-hydroxypiperazin-1-yl)-2,3-dihydro-1,4-benzodioxine;
7-chloro-5-(4-hydroxypiperazin-1-yl)-2,3-dihydro-1,4-benzodioxine;
8-chloro-5-(4-hydroxypiperazin-1-yl)-2,3-dihydro-1,4-benzodioxine;
5-(2-aminopiperazin-1-yl)-2-hydroxy-2,3-dihydro-1,4-benzodioxine;
5-(2-aminopiperazin-1-yl)-3-hydroxy-2,3-dihydro-1,4-benzodioxine;
5-(2-aminopiperazin-1-yl)-6-hydroxy-2,3-dihydro-1,4-benzodioxine;
5-(2-aminopiperazin-1-yl)-7-hydroxy-2,3-dihydro-1,4-benzodioxine;
5-(2-aminopiperazin-1-yl)-8-hydroxy-2,3-dihydro-1,4-benzodioxine;
5-(3-aminopiperazin-1-yl)-2-hydroxy-2,3-dihydro-1,4-benzodioxine;
5-(3-aminopiperazin-1-yl)-3-hydroxy-2,3-dihydro-1,4-benzodioxine;
5-(3-aminopiperazin-1-yl)-6-hydroxy-2,3-dihydro-1,4-benzodioxine;
5-(3-aminopiperazin-1-yl)-7-hydroxy-2,3-dihydro-1,4-benzodioxine;
5-(3-aminopiperazin-1-yl)-8-hydroxy-2,3-dihydro-1,4-benzodioxine;
5-(4-aminopiperazin-1-yl)-2-hydroxy-2,3-dihydro-1,4-benzodioxine;
5-(4-aminopiperazin-1-yl)-3-hydroxy-2,3-dihydro-1,4-benzodioxine;
5-(4-aminopiperazin-1-yl)-6-hydroxy-2,3-dihydro-1,4-benzodioxine;
5-(4-aminopiperazin-1-yl)-7-hydroxy-2,3-dihydro-1,4-benzodioxine;
5-(4-aminopiperazin-1-yl)-8-hydroxy-2,3-dihydro-1,4-benzodioxine;
2-amino-5-(2-hydroxypiperazin-1-yl)-2,3-dihydro-1,4-benzodioxine;
3-amino-5-(2-hydroxypiperazin-1-yl)-2,3-dihydro-1,4-benzodioxine;
6-amino-5-(2-hydroxypiperazin-1-yl)-2,3-dihydro-1,4-benzodioxine;
7-amino-5-(2-hydroxypiperazin-1-yl)-2,3-dihydro-1,4-benzodioxine;
8-amino-5-(2-hydroxypiperazin-1-yl)-2,3-dihydro-1,4-benzodioxine;
2-amino-5-(3-hydroxypiperazin-1-yl)-2,3-dihydro-1,4-benzodioxine;
3-amino-5-(3-hydroxypiperazin-1-yl)-2,3-dihydro-1,4-benzodioxine;
6-amino-5-(3-hydroxypiperazin-1-yl)-2,3-dihydro-1,4-benzodioxine;
7-amino-5-(3-hydroxypiperazin-1-yl)-2,3-dihydro-1,4-benzodioxine;
8-amino-5-(3-hydroxypiperazin-1-yl)-2,3-dihydro-1,4-benzodioxine;
2-amino-5-(4-hydroxypiperazin-1-yl)-2,3-dihydro-1,4-benzodioxine;
3-amino-5-(4-hydroxypiperazin-1-yl)-2,3-dihydro-1,4-benzodioxine;
6-amino-5-(4-hydroxypiperazin-1-yl)-2,3-dihydro-1,4-benzodioxine;
7-amino-5-(4-hydroxypiperazin-1-yl)-2,3-dihydro-1,4-benzodioxine; and
8-amino-5-(4-hydroxypiperazin-1-yl)-2,3-dihydro-1,4-benzodioxine.

Examples of pharmaceutically acceptable salts include, but are not limited to, salts or acid addition salts prepared by mixing a solution of the 5-HT_{1A/1B} receptor agonist or the compound of formula (I) with a solution of an acid selected from the group consisting of phosphoric acid, sulfuric acid, nitric acid, diphosphoric acid, bicarbonic acid, carbonic acid, clavulanic acid, isethionic acid, boric acid, halides, hydrohalic acids, nitric acid, acetic acid, succinic acid, lactic acid, tartaric acid, lactobionic acid, lauric acid, mandelic acid, malic acid, citric acid, fumaric acid, maleic acid, oleic acid, oxalic acid, ascorbic acid, nicotinic acid, benzoic acid, mesylic acid, salicylic acid, stearic acid, tannic acid, tosic acid, valeric acid, methanesulfonic acid, ethanesulfonic acid, benzenesulfonic acid, p-toluenesulfonic acid, 2-ethanedisulfonic acid, and naphthalenesulfonic acid.

Furthermore, when the chemical structure of the 5-HT_{1A/1B} receptor agonist or the compound of formula (I) has an acidic moiety, examples of the pharmaceutically acceptable salts include, but are not limited to, alkali metal salts such as sodium or potassium salts; alkaline earth metal salts such as calcium or magnesium salts; and salts formed with suitable organic ligands, such as quaternary ammonium salts. Specific examples of the pharmaceutically acceptable salts of the compound include, but are not limited to, acetate, benzenesulfonate, benzoate, bicarbonate, bisulfate, bitartrate, borate, bromide, calcium, carbonate, chloride, clavulanate, citrate, dihydrochloride, fumarate, gluconate, glutamate, hydrobromide, hydrochloride, hydroxynaphthoate, iodide, isethionate, lactate, lactobionate, laurate, maleate, mandelate, mesylate, methylbromide, methylnitrate, methylsulfate, nitrate, N-methylglucamine ammonium salt, oleate, oxalate, phosphate/diphosphate, salicylate, stearate, sulfate, succinate, tannate, tartrate, toluenesulfonate, triethiodide, and valerate.

The present disclosure encompasses within its scope prodrugs of the 5-HT_{1A/1B} receptor agonist or the compound of formula (I). A prodrug is an inactive derivative of the compound that can be readily converted in vivo into a desired compound. Conventional methods for selecting and producing suitable prodrug derivatives are described, for example, in "Design of Prodrugs" (Editor: H. Bundgaard, Elsevier, 1985). This publication is incorporated herein by reference.

When the structure of the 5-HT_{1A/1B} receptor agonist or the compound of formula (I) includes at least one chiral center, such a compound may exist as a racemic mixture or an enantiomer. Such an isomer and mixture thereof fall within the scope of the present disclosure. Furthermore, the present disclosure also encompasses crystalline forms, crystals, crystalline solids, and polymorphs of the 5-HT_{1A/1B} receptor agonist or the compound of formula (I), if they exist. In addition, solvates and hydrates of the 5-HT_{1A/1B} receptor agonist or the compound of formula (I) are within the scope of the present disclosure. The crystalline forms, crystals, crystalline solids, and polymorphs of the 5-HT_{1A/1B} receptor agonist or the compound of formula (I) can be obtained, for example, by the methods described in International Publication No. WO2006/037043 pamphlet and International Publication No. WO2006/036874 pamphlet, the contents of which are incorporated herein by reference.

The 5-HT_{1A/1B} receptor agonist is commercially available. Whether a given compound is a 5-HT_{1A/1B} receptor agonist can be readily determined by methods well known to those skilled in the art. The compound of formula (I) is also commercially available. The compound of formula (I) can be synthesized using methods based on organic synthetic chemistry. See, for example, Japanese Unexamined Patent Application Publication No. S61-152655 and U.S. Patent No. 5424313. These publications are incorporated herein by reference.

The 5-HT_{1A} and 5-HT_{1B} receptors are classified as G protein-coupled receptors (GPCR), and screening methods for ligands thereof have already been established and are commercially available (see, for example, Ryo Shimizu et al., Proceedings of the Symposium on Chemoinformatics, 29th Symposium on Information Chemistry, Niigata, JL1, "Application of active learning to screening of GPCR ligand"; and INSIGHTS INTO GPCR DRUG DISCOVERY & DEVELOPMENT, Exploring GPCR-Ligand Interactions and Signaling Pathways with Binding and Functional Assays, Eurofins, DiscoverX, 2022, particularly page 64; these publications are incorporated herein by reference). In addition, GPCR profiling and screening services are provided by many companies, such as Cosmo Bio Co., Ltd. Accordingly, a person skilled in the art can easily screen for ligands of the 5-HT_{1A/1B} receptors.

### (4. Pharmaceutical Composition)

The present disclosure provides a pharmaceutical composition containing 5-HT_{1A/1B} receptor agonist. In one embodiment, the pharmaceutical composition of the present disclosure contains a therapeutically effective amount of 5-HT_{1A/1B} receptor agonist. The pharmaceutical composition of the present disclosure may further contain a pharmaceutically acceptable carrier or excipient. The dosage, administration method, administration route, administration frequency, administration interval, order of administration, timing of administration, and the like of the pharmaceutical composition of the present disclosure are not particularly limited. They can be determined by a physician or other medical experts based on the condition of the subject.

The pharmaceutical composition of the present disclosure can be used to treat, prevent, or manage the disorders described in the present specification. The pharmaceutical composition of the present disclosure can be used in the manufacture of a medicament for treating, preventing, or managing the disorders described in the present specification. The pharmaceutical composition of the present disclosure can be used to treat, prevent, or manage the subjects described in the present specification. The pharmaceutical composition of the present disclosure can be used in the manufacture of a medicament for treating, preventing, or managing the subjects described in the present specification.

The pharmaceutical composition of the present disclosure can be used in the treatment, prevention, or management methods described in the present specification.

In one embodiment, the pharmaceutical composition of the present disclosure is used to treat, prevent, or manage BIND, a psychiatric disorder associated with jaundice, or a psychiatric disorder caused by UGT1A1 gene mutation in a subject. In one embodiment, the pharmaceutical composition of the present disclosure is used in the manufacture of a medicament for treating, preventing, or managing BIND, a psychiatric disorder associated with jaundice, or a psychiatric disorder caused by UGT1A1 gene mutation in a subject.

In one embodiment, the pharmaceutical composition of the present disclosure is used to treat one or more (e.g., one, two, or three) disorders selected from the group consisting of BIND, a psychiatric disorder associated with jaundice, and a psychiatric disorder caused by UGT1A1 gene mutation in a subject. In one embodiment, the pharmaceutical composition of the present disclosure is used to prevent one or more (e.g., one, two, or three) disorders selected from the group consisting of BIND, a psychiatric disorder associated with jaundice, and a psychiatric disorder caused by UGT1A1 gene mutation in a subject. In one embodiment, the pharmaceutical composition of the present disclosure is used to manage one or more (e.g., one, two, or three) disorders selected from the group consisting of BIND, a psychiatric disorder associated with jaundice, and a psychiatric disorder caused by UGT1A1 gene mutation in a subject.

In one embodiment, the pharmaceutical composition of the present disclosure is used to treat, prevent, or manage BIND in a subject. In one embodiment, the pharmaceutical composition of the present disclosure is used to treat, prevent, or manage a psychiatric disorder associated with jaundice in a subject. In one embodiment, the pharmaceutical composition of the present disclosure is used to treat, prevent, or manage a psychiatric disorder caused by UGT1A1 gene mutation in a subject.

In some embodiments, the pharmaceutical composition of the present disclosure is used in the treatment, prevention, or management methods for a psychiatric disorder in a subject.

The pharmaceutical composition of the present disclosure can be used to treat, prevent, or manage the subjects. In some embodiments, the subject has a high bilirubin level in the biological sample. In some embodiments, the subject has a high bilirubin level in the biological sample compared to that of a healthy subject. In some embodiments, the subject has BIND. In some embodiments, the subject has jaundice. In some embodiments, the subject has UGT1A1 gene mutation. In some embodiments, the subject has one or more (e.g., one, two, three, four, or five) conditions selected from the group consisting of a psychiatric disorder, BIND, UGT1A1 gene mutation, jaundice, and a high bilirubin level in the biological sample, for example, a bilirubin level higher than that of a healthy subject.

The pharmaceutical composition of the present disclosure can be formulated for oral, parenteral (e.g., intravenous, intramuscular, or subcutaneous), intranasal, sublingual or rectal administration, or for administration by inhalation or spraying. For example, the pharmaceutical composition of the present disclosure may be in the form of a tablet, pill, capsule (e.g., sustained-release or delayed-release formulation), powder, granule, elixir, tincture, syrup, emulsion, sterile parenteral aqueous solution or suspension, aerosol or liquid spray, drop, ampoule, auto-injector, or suppository. The pharmaceutical composition of the present disclosure can be formulated by usual methods, such as those described in Remington's Pharmaceutical Sciences (edited by Gennaro, Mack Publishing Co., Easton PA, 1990), which is incorporated herein by reference.

A pharmaceutically acceptable carrier or excipient is a nontoxic, inert solid, semi-solid, or liquid substance. Examples of pharmaceutically acceptable carriers or excipients include, for example, stabilizers, inert diluents, fillers, expanders, disintegrants, disintegration inhibitors, suspending agents, buffering agents, isotonizing agents, chelating agents, pH adjusters, surfactants, encapsulating agents, binders, preservatives, antioxidants, lubricants, humectants, adsorbents, glidants, and any other formulation aids. Specific examples of pharmaceutically acceptable carriers or excipients include, but are not limited to, water, alcohols (e.g., ethanol), solvents (e.g., dimethyl sulfoxide), saline solutions (e.g., physiological saline), or mixed solutions thereof; inorganic salts such as sodium chloride; acids such as boric acid, phosphoric acid, acetic acid, citric acid, ε-aminocaproic acid, glutamic acid, and edetic acid, or their corresponding salts, for example, alkali metal salts or alkaline earth metal salts such as sodium, potassium, calcium, or magnesium salts; monosaccharides such as glucose (dextrose), mannose, galactose, or fructose; sugar alcohols such as mannitol, inositol, or xylitol; disaccharides such as sucrose, lactulose, lactose, maltose, trehalose, or cellobiose; polysaccharides such as dextrin, dextran, cellulose, hyaluronic acid, or chondroitin sulfate; starches such as corn starch and potato starch; celluloses such as cellulose, methylcellulose, ethylcellulose, hydroxyethyl cellulose, hydroxypropyl cellulose, hydroxypropyl methylcellulose, carboxymethylcellulose sodium, cellulose acetate, or derivatives thereof; human serum albumin; L-amino acids such as glycine, lysine, asparagine, arginine, glutamine, cysteine, aspartic acid, or glutamic acid; tragacanth; gelatin; talc; cacao butter or wax; waxes or beeswax; oils such as peanut oil, cottonseed oil, safflower oil, sesame oil, olive oil, corn oil, soybean oil, or hydrogenated oils; glycols such as ethylene glycol or propylene glycol; esters such as ethyl oleate or ethyl laurate; agar; potassium hydroxide, sodium hydroxide, magnesium hydroxide, or aluminum hydroxide; alginic acid; isotonic saline solutions; Ringer's solution; ethyl alcohol; phosphate buffer; and sodium lauryl sulfate or magnesium stearate. The pharmaceutically acceptable carrier or excipient can be appropriately selected, for example, depending on the type of dosage form and the compound to be used.

The amounts of the 5-HT_{1A/1B} receptor agonist, the second therapeutic agent, and a pharmaceutically acceptable carrier or excipient contained in the pharmaceutical composition of the present disclosure are not particularly limited, as long as they are effective for treating, preventing, or managing the disorder. In one embodiment, the amount of the 5-HT_{1A/1B} receptor agonist contained in the pharmaceutical composition of the present disclosure is a therapeutically effective amount. The amounts of the 5-HT_{1A/1B} receptor agonist, the second therapeutic agent, and the pharmaceutically acceptable carrier or excipient contained in the pharmaceutical composition of the present disclosure may be determined based on various factors, such as the type of compound used; the age, body weight, health condition, gender, and diet of the mammal subjected to the administration; the number and route of administrations; the treatment period; and other agents used concurrently. The 5-HT_{1A/1B} receptor agonist is contained in the pharmaceutical composition of the present disclosure, for example, in an amount of about 0.01 mg to about 100 mg, about 0.1 mg to about 50 mg, about 0.5 mg to about 50 mg, about 1 mg to about 25 mg, about 2.5 mg to about 25 mg, about 5 mg to about 25 mg, or about 10 mg to about 20 mg. In some embodiments, the pharmaceutical composition of the present disclosure is used so that the 5-HT_{1A/1B} receptor agonist is administered to a subject in an amount of about 0.0001 mg/kg (body weight) to about 50 mg/kg (body weight), about 0.001 mg/kg (body weight) to about 50 mg/kg (body weight), about 0.01 mg/kg (body weight) to about 50 mg/kg (body weight), about 0.1 mg/kg (body weight) to about 50 mg/kg (body weight), about 0.1 mg/kg (body weight) to about 40 mg/kg (body weight), about 0.1 mg/kg (body weight) to less than about 34 mg/kg (body weight), about 0.1 mg/kg (body weight) to about 30 mg/kg (body weight), about 0.1 mg/kg (body weight) to about 20 mg/kg (body weight), about 0.5 mg/kg (body weight) to about 10 mg/kg (body weight), or about 1 mg/kg (body weight) to about 10 mg/kg (body weight) per day or at each dose. The amount of the pharmaceutically acceptable carrier or excipient contained in the pharmaceutical composition of the present disclosure may be about 1 to about 99% by weight, about 5 to about 90% by weight, about 10 to about 80% by weight, about 20 to about 70% by weight, about 30 to about 60% by weight, or about 40 to about 50% by weight of the pharmaceutical composition of the present disclosure. The 5-HT_{1A/1B} receptor agonist may be administered such that a single daily dose is administered at once, or the entire daily dose is administered in divided doses, e.g., 2, 3 or 4 times per day.

The pharmaceutical composition of the present disclosure can be formulated for oral or parenteral administration, for example, intravenous, subcutaneous, enteral, intraperitoneal, or intramuscular administration. The formulation may contain a 5-HT_{1A/1B} receptor agonist, and optionally, a carrier or excipient. In the formulation, the 5-HT_{1A/1B} receptor agonist may be uniformly present. Examples of the formulations include those of a solid dosage form such as tablets, pills, powders, fine powders, granules, or capsules, or those of a liquid dosage form such as a sterile aqueous solution, sterile non-aqueous solution, suspension, milky emulsion, syrup, emulsion, and elixir. The type of dosage form is not particularly limited and may be appropriately selected depending on the administration route. The pharmaceutical composition of the present disclosure may be administered, for example, orally as a tablet in the form of solid dosage form, or intravenously using a syringe or catheter in the form of a liquid dosage form.

In accordance with conventional pharmaceutical compounding techniques, a solid formulation can be prepared by combining the active ingredient with one or more substances selected from the group consisting of excipients, binders, disintegrants, surfactants, disintegration inhibitors, absorption enhancers, humectants, adsorbents, and lubricants. Examples of binders include, but are not limited to, natural saccharides such as starch, gelatin, glucose, or β-lactose, natural or synthetic gums such as acacia, tragacanth, or sodium alginate, carboxymethylcellulose, polyethylene glycol, and wax. Examples of disintegrants include, but are not limited to, starch, methylcellulose, agar, bentonite, and xanthan gum. Examples of lubricants include, but are not limited to, sodium oleate, sodium stearate, magnesium stearate, sodium benzoate, sodium acetate, and sodium chloride. The solid formulation may additionally contain, as needed, colorants, preservatives, flavors, flavoring agents, sweeteners, and the like. Furthermore, the solid formulation may be, for example, a sugar-coated tablet, gelatin-coated tablet, enteric-coated tablet, or film-coated tablet, and may also be in the form of a double-layered or multilayered tablet. Capsules can be prepared in an usual manner by mixing the active component with various pharmaceutically acceptable carriers and filling hard gelatin capsules, soft capsules, or the like with the mixture. The concentration of the active ingredient in the solid formulation is not particularly limited and may be, for example, about 1 % by weight to about 99 % by weight, about 20 % by weight to about 80 % by weight, about 30 % by weight to about 60 % by weight, or about 40 % by weight to about 50 % by weight. The solid formulation may contain a single dose or a divided dose of the active ingredient.

The liquid formulation may be prepared by mixing the active ingredient with an inert diluent and the like in accordance with conventional pharmaceutical compounding techniques. In addition, optionally, one or more substances selected from the group consisting of solubilizers, buffers, isotonizing agents, suspending agents, surfactants, antioxidants, and preservatives may be added. Furthermore, the liquid formulation may be prepared by initially formulating and storing the pharmaceutical composition of the present disclosure as freeze-dried powder and dissolving or suspending it in an inert diluent or the like immediately prior to use. The liquid formulation may additionally contain, as needed, colorants, preservatives, flavors, flavoring agents, sweeteners, and the like. The concentration of the active ingredient in the liquid formulation is not particularly limited and may be, for example, about 1 % by weight to about 99 % by weight, about 20 % by weight to about 80 % by weight, about 30 % by weight to about 60 % by weight, or about 40 % by weight to about 50 % by weight. The liquid formulation may contain a single dose or a divided dose of the active ingredient.

The pharmaceutical composition of the present disclosure may further comprise a second therapeutic agent. The pharmaceutical composition of the present disclosure can be used such that the 5-HT_{1A/1B} receptor agonist is administered to a subject simultaneously, concurrently, or sequentially with the second therapeutic agent. In the case of sequential administration, either the 5-HT_{1A/1B} receptor agonist or the second therapeutic agent may be administered first. The second therapeutic agent is, for example, a drug for treating, preventing, or managing psychiatric disorders, such as known anti-psychotic agents, anti-schizophrenic agents, or anti-attention deficit-hyperactivity-disorder (ADHD) agent. In some embodiments, the second therapeutic agent is one or more therapeutic agents selected from the group consisting of cholinesterase inhibitors, acetylcholinesterase inhibitors, butyrylcholinesterase inhibitors, muscarinic receptor agonists, glutamatergic agonist-antagonists, cholinergic agents, carnitine acetyltransferase stimulators, acetylcholine release stimulators, choline uptake stimulators, nicotinic acetylcholine receptor agonists, 5-HT6 antagonists, 5-HT6 inverse agonists, antidyskinetic agents, antidystonic agents, antimyoclonic agents, antitremor agents, selective serotonin reuptake inhibitors (SSRI), serotonin-noradrenaline reuptake inhibitors (SNRI), monoamine oxidase inhibitors (MAOI), tricyclic antidepressants (TCA), antipsychotics, norepinephrine reuptake inhibitors, dopaminergic agonists, hypnotic agents, antimanic agents, and antiphobic agents. In some embodiments, the second therapeutic agent is one or more therapeutic agents selected from the group consisting of SB-742457, SB-271046, SB-399885, SB-357134, SB-258585, RO-436854, RO-0406790, and RO-65-7674. In some embodiments, the second therapeutic agent is one or more therapeutic agents selected from the group consisting of levodopa, bromocriptine, pergolide, ephedrine sulfate, pemoline, mazindol, d,1-α-methylphenethylamine, methylphenidate, pramipexole, modafinil, and ropinirole. The dosage, administration method, administration route, administration frequency, administration interval, order of administration, timing of administration, and the like of the second therapeutic agent are not particularly limited and can be determined by a physician or other medical experts based on the condition of the subject. The dosage of the second therapeutic agent may be an amount effective for treating, preventing, or managing psychiatric disorders.

### (5. Treatment, Prevention, or Management Methods)

The present disclosure provides a method for treating, preventing, or managing a disorder or condition. The method comprises a step of administering a 5-HT_{1A/1B} receptor agonist or the pharmaceutical composition of the present disclosure. In some embodiments, the method of the present disclosure comprises a step of administering a therapeutically effective amount of a 5-HT_{1A/1B} receptor agonist to a subject.

The method of the present disclosure can be used to treat, prevent, or manage the disorders described in the present specification. The method of the present disclosure can be used to treat, prevent, or manage the subjects described in the present specification.

In one embodiment, the present disclosure provides a method for treating, preventing, or managing BIND, a psychiatric disorder associated with jaundice, or a psychiatric disorder caused by UGT1A1 gene mutations in a subject in need thereof. The method comprises a step of administering to the subject a therapeutically effective amount of a 5-HT_{1A/1B} receptor agonist or a therapeutically effective amount of the pharmaceutical composition of the present disclosure.

In one embodiment, the method of the present disclosure is a method for treating one or more (e.g., one, two, or three) disorders selected from the group consisting of BIND, a psychiatric disorder associated with jaundice, and a psychiatric disorder caused by UGT1A1 gene mutation in a subject in need thereof. The method comprises a step of administering a therapeutically effective amount of a 5-HT_{1A/1B} receptor agonist to the subject. In one embodiment, the method of the present disclosure is a method for preventing one or more (e.g., one, two, or three) disorders selected from the group consisting of BIND, a psychiatric disorder associated with jaundice, and a psychiatric disorder caused by UGT1A1 gene mutation in a subject in need thereof. The method comprises a step of administering a therapeutically effective amount of a 5-HT_{1A/1B} receptor agonist to the subject. In one embodiment, the method of the present disclosure is a method for managing one or more (e.g., one, two, or three) disorders selected from the group consisting of BIND, a psychiatric disorder associated with jaundice, and a psychiatric disorder caused by UGT1A1 gene mutation in a subject in need thereof. The method comprises a step of administering a therapeutically effective amount of a 5-HT_{1A/1B} receptor agonist to the subject.

In one embodiment, the method of the present disclosure is a method for treating, preventing, or managing BIND in a subject in need thereof. The method comprises a step of administering a therapeutically effective amount of a 5-HT_{1A/1B} receptor agonist to the subject. In one embodiment, the method of the present disclosure is a method for treating, preventing, or managing a psychiatric disorder associated with jaundice in a subject. The method comprises a step of administering a therapeutically effective amount of a 5-HT_{1A/1B} receptor agonist to the subject. In one embodiment, the method of the present disclosure is a method for treating, preventing, or managing a psychiatric disorder caused by UGT1A1 gene mutations in a subject. The method comprises a step of administering a therapeutically effective amount of a 5-HT_{1A/1B} receptor agonist to the subject.

In some embodiments, the method of the present disclosure is a method for treating, preventing, or managing a psychiatric disorder in a subject in need thereof. The method comprises a step of administering a therapeutically effective amount of a 5-HT_{1A/1B} receptor agonist to the subject.

In some embodiments, the method of the present disclosure further comprises a step of collecting a biological sample from a subject. The method of collecting a biological sample may be a method known to those skilled in the art. For example, blood can be collected from the subject using a syringe or similar devices. The biological sample is preferably blood or urine.

In some embodiments, the method of the present disclosure further comprises a step of measuring a bilirubin level in a biological sample collected from the subject. The measurement of the bilirubin level may be carried out using methods known to those skilled in the art. Examples of the methods include a method using total bilirubin blood test kit CicaLiquid D-BIL (KANTO KAGAKU), a method using vanadic acid (see Japanese Journal of Clinical Chemistry 22: 116-122, 1993, this publication is incorporated herein by reference), a method using direct bilirubin kit Aqua-auto Kainos D-BIL reagent (KAINOS Laboratories, Inc.), or a method using total bilirubin measurement "serotec" T-BIL (serotec Co,.Ltd).

In some embodiments, the method of the present disclosure comprises a step of determining whether the subject has BIND. Whether the subject has BIND can be determined by methods known to those skilled in the art. For example, the presence or absence of BIND in a subject can be determined by examining the bilirubin level (total bilirubin level, direct bilirubin level, or indirect bilirubin level) in a biological sample derived from the subject, a UGT1A1 gene mutation, or a combination of two or more, three or more, or four of these factors. For example, if the blood bilirubin level is increased in the subject, such as when the blood bilirubin level in the subject is higher than that of a healthy subject, it can be determined that the subject has BIND.

In some embodiments, the method of the present disclosure comprises a step of determining whether the subject has jaundice. Whether the subject has jaundice can be determined by methods known to those skilled in the art. For example, constitutional hyperbilirubinemia can be tested by a blood test, in which the indirect bilirubin level in the blood is measured. For example, if the blood indirect bilirubin level is increased in the subject, such as when the blood bilirubin level in the subject is higher than that of a healthy subject, it can be determined that the subject has jaundice.

In some embodiments, the method of the present disclosure further comprises a step of determining whether the subject has a UGT1A1 mutation. Whether the subject has a UGT1A1 gene mutation can be determined by methods known to those skilled in the art. For example, the Invader (registered trademark) UGT1A1 assay (Sekisui Medical Co., Ltd.) can be used as a reagent for determining UGT1A1 gene polymorphism.

In some embodiments, the method of the present disclosure comprises a step of determining whether the subject has a psychiatric disorder. Whether the subject has a psychiatric disorder can be determined by methods known to those skilled in the art. For example, the determination can be made using diagnostic tests for various psychiatric disorders, interviews, electroencephalography, and CT scans, or a combination of one or more of these methods.

The method of the present disclosure can be used to treat, prevent, or manage the subjects. In some embodiments, the subject has a psychiatric disorder. In some embodiments, the subject has BIND. In some embodiments, the subject has jaundice. In some embodiments, the subject has UGT1A1 gene mutation. In some embodiments, the subject has a high bilirubin level in the biological sample. In some embodiments, the subject has a high bilirubin level in the biological sample compared to that of a healthy subject. In some embodiments, the subject has one or more (e.g., one, two, three, four, or five) conditions selected from the group consisting of a psychiatric disorder, BIND, jaundice, UGT1A1 gene mutation, and a high bilirubin level in the biological sample, for example, a bilirubin level higher than that of a healthy subject.

In a specific embodiment, the method of the present disclosure is a method for treating, preventing, or managing BIND or a psychiatric disorder in a subject in need thereof, wherein the method comprises steps of:
(a) obtaining a biological sample from the subject;
(b) measuring a bilirubin level in the biological sample obtained in the step (a); and
(c) administering to the subject a therapeutically effective amount of a 5-HT_{1A/1B} receptor agonist or a therapeutically effective amount of the pharmaceutical composition of the present disclosure, when the bilirubin level measured in the step (b) is higher than the bilirubin level of a healthy subject, or when it is determined that the subject has jaundice based on the bilirubin level measured in the step (b). The psychiatric disorder may include a psychiatric disorder associated with jaundice and/or a psychiatric disorder caused by UGT1A1 gene mutations.

In a specific embodiment, the method of the present disclosure is a method for treating, preventing, or managing BIND or a psychiatric disorder in a subject in need thereof, wherein the method comprises steps of:
(a) determining whether the subject has a UGT1A1 gene mutation; and
(b) administering to the subject a therapeutically effective amount of a 5-HT_{1A/1B} receptor agonist or a therapeutically effective amount of the pharmaceutical composition of the present disclosure, when it is determined that the subject has a UGT1A1 gene mutation in the step (a). The psychiatric disorder may include a psychiatric disorder associated with jaundice and/or a psychiatric disorder caused by UGT1A1 gene mutations.

In some embodiments, the method of the present disclosure further comprises a step of determining whether the subject has a disorder or condition described in the present specification, or whether it is possible that the subject has a disorder or condition described in the present specification. In a specific embodiment, the method of the present disclosure further comprises a step of determining whether the subject has a psychiatric disorder, BIND, a psychiatric disorder associated with jaundice, or a psychiatric disorder caused by UGT1A1 gene mutation, or whether it is possible that the subject has a psychiatric disorder, BIND, a psychiatric disorder associated with jaundice, or a psychiatric disorder caused by UGT1A1 gene mutation. In some embodiments, the method of the present disclosure further comprises a step of diagnosing a psychiatric symptom or mental state of the subject.

The dosage, administration method, administration route, administration frequency, administration interval, order of administration, timing of administration, and the like of the 5-HT_{1A/1B} receptor agonist or the pharmaceutical composition of the present disclosure are not particularly limited. They can be determined by a physician or other medical experts based on the condition of the subject. The dosage of the 5-HT_{1A/1B} receptor agonist or the pharmaceutical composition of the present disclosure may be an amount effective for treating, preventing, or managing BIND, psychiatric disorders associated with jaundice, or psychiatric disorders caused by UGT1A1 gene mutations. The dosage is, for example, about 0.01 mg to about 100 mg, about 0.1 mg to about 50 mg, about 0.5 mg to about 50 mg, about 1 mg to about 25 mg, about 2.5 mg to about 25 mg, about 5 mg to about 25 mg, or about 10 mg to about 20 mg, per day or at each dose. In some embodiments, the 5-HT_{1A/1B} receptor agonist is administered to a subject in an amount of about 0.0001 mg/kg (body weight) to about 50 mg/kg (body weight), about 0.001 mg/kg (body weight) to about 50 mg/kg (body weight), about 0.01 mg/kg (body weight) to about 50 mg/kg (body weight), about 0.1 mg/kg (body weight) to about 50 mg/kg (body weight), about 0.1 mg/kg (body weight) to about 40 mg/kg (body weight), about 0.1 mg/kg (body weight) to less than about 34 mg/kg (body weight), about 0.1 mg/kg (body weight) to about 30 mg/kg (body weight), about 0.1 mg/kg (body weight) to about 20 mg/kg (body weight), about 0.5 mg/kg (body weight) to about 10 mg/kg (body weight), or about 1 mg/kg (body weight) to about 10 mg/kg (body weight) per day or at each dose. Examples of administration routes include, but are not limited to, oral or parenteral administration, for example, intravenous, subcutaneous, enteral, intraperitoneal, or intramuscular administration. The administration frequency is, for example, once a day, twice a day, three times a day, or once every two days. The administration interval is 6, 8, 12, 18, or 24 hours, 2, 3, 4, or 5 days.

The treatment, prevention, or management methods of the present disclosure may further comprise a step of administering to the subject a therapeutically effective amount of a second therapeutic agent. The second therapeutic agent is, for example, a drug for treating, preventing, or managing psychiatric disorders, such as known anti-psychotic agents, anti-schizophrenic agents, or anti-attention deficit-hyperactivity-disorder (ADHD) agent. In one embodiment, the treatment, prevention, or management methods of the present disclosure comprises a step of administering a 5-HT_{1A/1B} receptor agonist simultaneously, concurrently, or sequentially with the second therapeutic agent. In some embodiments, the second therapeutic agent is one or more therapeutic agents selected from the group consisting of cholinesterase inhibitors, acetylcholinesterase inhibitors, butyrylcholinesterase inhibitors, muscarinic receptor agonists, glutamatergic agonist-antagonists, cholinergic agents, carnitine acetyltransferase stimulators, acetylcholine release stimulators, choline uptake stimulators, nicotinic acetylcholine receptor agonists, 5-HT6 antagonists, 5-HT6 inverse agonists, antidyskinetic agents, antidystonic agents, antimyoclonic agents, antitremor agents, selective serotonin reuptake inhibitors (SSRI), serotonin-noradrenaline reuptake inhibitors (SNRI), monoamine oxidase inhibitors (MAOI), tricyclic antidepressants (TCA), antipsychotics, norepinephrine reuptake inhibitors, dopaminergic agonists, hypnotic agents, antimanic agents, and antiphobic agents. In some embodiments, the second therapeutic agent is one or more therapeutic agents selected from the group consisting of SB-742457, SB-271046, SB-399885, SB-357134, SB-258585, RO-436854, RO-0406790, and RO-65-7674. In some embodiments, the second therapeutic agent is one or more therapeutic agents selected from the group consisting of levodopa, bromocriptine, pergolide, ephedrine sulfate, pemoline, mazindol, d,1-α-methylphenethylamine, methylphenidate, pramipexole, modafinil, and ropinirole. The dosage, administration method, administration route, administration frequency, administration interval, order of administration, timing of administration, and the like of the second therapeutic agent are not particularly limited and can be determined by a physician or other medical experts based on the condition of the subject. The dosage of the second therapeutic agent may be an amount effective for treating, preventing, or managing psychiatric disorders.

In one embodiment, the treatment, prevention, or management methods of the present disclosure further comprises a step of identifying whether the subject has BIND, a psychiatric disorder associated with jaundice, or a psychiatric disorder caused by UGT1A1 gene mutations. In other embodiments, the treatment, prevention, or management methods of the present disclosure further comprises a step of identifying whether the subject has jaundice, and/or whether the subject has a UGT1A1 gene mutation.

### (6. Kit)

The present disclosure provides a kit including a 5-HT_{1A/1B} receptor agonist or the pharmaceutical composition of the present disclosure. In some embodiments, the kit comprises a therapeutically effective amount of a 5-HT_{1A/1B} receptor agonist or a therapeutically effective amount of the pharmaceutical composition of the present disclosure.

The kit of the present disclosure may further comprises a reagent for measuring the bilirubin level in a biological sample and/or a reagent for measuring bilirubin UDP-glucuronosyltransferase gene mutation. In some embodiments, the bilirubin level is a total bilirubin level, indirect bilirubin level, or direct bilirubin level. In some embodiments, the bilirubin level is one or more (e.g., one, two, or three) selected from the group consisting of total bilirubin level, indirect bilirubin level, and direct bilirubin level.

Examples of the reagents for measuring the bilirubin level in a biological sample include, but are not limited to, reagents used for total bilirubin blood test kit CicaLiquid D-BIL (KANTO KAGAKU), a method using vanadic acid (see Japanese Journal of Clinical Chemistry 22: 116-122, 1993, this publication is incorporated herein by reference), direct bilirubin kit Aqua-auto Kainos D-BIL reagent (KAINOS Laboratories, Inc.), or total bilirubin measurement "serotec" T-BIL (serotec Co,.Ltd). Specific examples of the reagents include, but are not limited to, bilirubin oxidase, vanadic acid, and sodium metavanadate. Indirect bilirubin level generally cannot be measured directly, and is typically calculated by subtracting the direct bilirubin level from the total bilirubin level.

Examples of the reagents for measuring the bilirubin UDP-glucuronosyltransferase gene mutation include, but are not limited to, the reagent used in the Invader (registered trademark) UGT1A1 assay (Sekisui Medical Co., Ltd.), which is used as a UGT1A1 genetic polymorphism determination reagent.

In some embodiments, the 5-HT_{1A/1B} receptor agonist or the pharmaceutical composition of the present disclosure is enclosed in a container such as an ampule or vial. Accordingly, in some embodiments, the kit of the present disclosure comprises a container containing the 5-HT_{1A/1B} receptor agonist or the pharmaceutical composition of the present disclosure.

The kit of the present disclosure can be used to treat, prevent, or manage the disorders described in the present specification. The kit of the present disclosure can be used to treat, prevent, or manage the subjects described in the present specification. The kit of the present disclosure can be used in the treatment, prevention, or management methods described in the present specification. In a specific embodiment, the kit of the present disclosure is used to treat, prevent, or manage BIND, a psychiatric disorder associated with jaundice, or a psychiatric disorder caused by UGT1A1 gene mutation in a subject.

In some embodiments, the kit of the present disclosure may further comprises instructions describing how to use the 5-HT_{1A/1B} receptor agonist or the pharmaceutical composition of the present disclosure. In some embodiments, the kit of the present disclosure comprises instructions describing the treatment, prevention, or management methods described in the present specification. In a specific embodiment, the kit of the present disclosure comprises instructions describing the treatment, prevention, or management methods for BIND, a psychiatric disorder associated with jaundice, or a psychiatric disorder caused by UGT1A1 gene mutation. In some embodiments, the kit of the present disclosure further comprises a material describing data on bilirubin levels in biological samples from healthy subjects. In some embodiments, the kit of the present disclosure comprises a device for collecting biological samples from a subject, such as a container (e.g., a urine collection cup) or a syringe.

In one embodiment, the kit of the present disclosure is used to treat one or more (e.g., one, two, or three) disorders selected from the group consisting of BIND, a psychiatric disorder associated with jaundice, and a psychiatric disorder caused by UGT1A1 gene mutation in a subject. In one embodiment, the kit of the present disclosure is used to prevent one or more (e.g., one, two, or three) disorders selected from the group consisting of BIND, a psychiatric disorder associated with jaundice, and a psychiatric disorder caused by UGT1A1 gene mutation in a subject. In one embodiment, the kit of the present disclosure is used to manage one or more (e.g., one, two, or three) disorders selected from the group consisting of BIND, a psychiatric disorder associated with jaundice, and a psychiatric disorder caused by UGT1A1 gene mutation in a subject.

In one embodiment, the kit of the present disclosure is used to treat, prevent, or manage BIND in a subject. In one embodiment, the kit of the present disclosure is used to treat, prevent, or manage a psychiatric disorder associated with jaundice in a subject. In one embodiment, the kit of the present disclosure is used to treat, prevent, or manage a psychiatric disorder caused by UGT1A1 gene mutation in a subject.

In some embodiments, the kit of the present disclosure is used in the treatment, prevention, or management methods for a psychiatric disorder in a subject.

The kit of the present disclosure can be used to treat, prevent, or manage the subjects. In some embodiments, the subject has a psychiatric disorder. In some embodiments, the subject has BIND. In some embodiments, the subject has jaundice. In some embodiments, the subject has UGT1A1 gene mutation. In some embodiments, the subject has a high bilirubin level in the biological sample. In some embodiments, the subject has a high bilirubin level in the biological sample compared to that of a healthy subject. In some embodiments, the subject has one or more (e.g., one, two, three, four, or five) conditions selected from the group consisting of a psychiatric disorder, BIND, jaundice, UGT1A1 gene mutation, and a high bilirubin level in the biological sample, for example, a bilirubin level higher than that of a healthy subject.

### EXAMPLE

### (7. Examples)

Examples are described below. The following examples are provided to facilitate understanding of the present invention, and are not intended to limit the scope thereof.

### (Example 1)

### (Confirmation Using Companion Diagnostics)

Examples of companion diagnostics include reagents for measuring total bilirubin, indirect bilirubin, and direct bilirubin levels, and reagents for measuring UGT1A1 gene mutations. Therefore, using an enzymatic method employed in typical bilirubin testing, the plasma components of Gunn rats (9 to 10 weeks of age), which serve as BIND model rats, were analyzed. Gunn rats were further examined with regard to UGT1A1 gene mutation. For comparison, Wistar rats (9 to 10 weeks of age), which are normal rats, were similarly analyzed.

Table 1 shows the total bilirubin, direct bilirubin, and indirect bilirubin levels in the plasma of Gunn rats and Wistar rats. In the table, * indicates p < 0.05, ** indicates p < 0.01, and *** indicates p < 0.001 (Student's t-test).

### [Table 1]

**Table 1**

| | Gunn rat | Wistar rat |
|---|---|---|
| Total Bilirubin (mg/dL) | 4.07 ±0.27*** | 0.02±0.004 |
| Direct Bilirubin (mg/dL) | 1.53±0.12*** | N.D (below detection limit) |
| Indirect Bilirubin (mg/dL) | 2. 54±0.16*** | 0. 02±0.004 |

| | | |
|---|---|---|
| (average ± standard error) Measure 6 each of Gunn rats and Wistar rats | | |

The various bilirubin levels in the plasma were significantly higher in Gunn rats, confirming that BIND can be distinguished by measuring the various (total, direct, and indirect) bilirubin levels.

It has been reported that Gunn rats carry a mutation in the UGT1A1 gene. (lyanagi T, Watanabe T, Uchiyama Y. The 3-methylcholanthrene-inducible UDP-glucuronosyltransferase deficiency in the hyperbilirubinemic rat (Gunn rat) is caused by a -1 frameshift mutation. J Biol Chem. 1989 Dec 15;264(35):21302-7.) Based on this,
it can be regarded that the mutation in the UGT1A1 gene is also useful for distinguishing BIND.

Gunn rats may also serve as model animals for psychiatric disorders associated with jaundice and psychiatric disorders caused by UGT1A1 gene mutations (see Non-Patent Literatures 6 and 7). Therefore, examining the various bilirubin levels (total bilirubin, direct bilirubin, and indirect bilirubin levels) makes it possible to distinguish psychiatric disorders associated with jaundice and psychiatric disorders caused by UGT1A1 gene mutations. In addition, mutations in the UGT1A1 gene are also useful for distinguishing psychiatric disorders associated with jaundice and psychiatric disorders caused by UGT1A1 gene mutations.

### (Example 2)

### (Open Field Test)

Eltoprazine (E1357, TCI), which is one of the 5-HT_{1A/1B} receptor agonists, was dissolved in an arbitrary solvent. 0.3 mg/kg of eltoprazine was subcutaneously administered to jaundice model rats (Gunn rats) at 9 to 10 weeks of age. Further, as control groups, only a solvent was subcutaneously administered to Gunn rats and Wistar rats at 9 to 10 weeks of age. Further, as a comparison group, 8OH-DPAT (0415005, FUJIFILM Wako Pure Chemical Corporation), which is one of 5-HT_{1A} receptor agonists, was dissolved in an arbitrary solvent, and 1 mg/kg thereof was subcutaneously administered to Gunn rats. After placing each rat in its home cage for 60 minutes, an open field test was conducted for 60 minutes using the Scanet MV-40 (Melquest Ltd.). The spontaneous activity amount and the number of rearing behaviors were measured for each rat by counting how many times the rat crossed the center evenly spaced. The number of grooming behaviors was counted by visual inspection. The results are shown in Tables 2 to 4 and FIGs. 1 to 3.

Table 2 and FIG. 1 show a spontaneous activity amount of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of 8OH-DPAT (8OH-DPAT(j/j)). In the figure, ** indicates p < 0.01 (Dunnett's test comparing each group with the Wistar rats administered only with a solvent).

**[Table 2]**

| **Table 2** | **Spontaneous Activity Amount** | | |
|---|---|---|---|
| | | Average (count) | Standard Error |
| Vehicle (+/+) | | 5920 | 633 |
| Vehicle(j/j) | | 11727 | 2102 |
| Elto (j/j) | | 4640 | 522 |
| 80H-DPAT(j/j) | | 2681 | 153 |

Table 3 and FIG. 2 show the number of rearing behaviors of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of 8OH-DPAT (8OH-DPAT(j/j)). In the figure, ** indicates p < 0.01 (Dunnett's test comparing each group with the Wistar rats administered only with a solvent).

**[Table 3]**

| **Table 3** | **Number of Rearing Behaviors** | | |
|---|---|---|---|
| | | Average (times) | Standard Error |
| Vehicle (+/+) | | 72, 3 | 8. 7 |
| Vehicle (j/j) | | 119.1 | 11.5 |
| Elto (j/j) | | 61.5 | 9.6 |
| 8OH-DPAT (j/j) | | 0.3 | 0.3 |

Table 4 and FIG. 3 show the number of grooming behaviors of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of 8OH-DPAT (8OH-DPAT(j/j)). In the figure, ** indicates p < 0.01 (Dunnett's test comparing each group with the Wistar rats administered only with a solvent).

**[Table 4]**

| **Table 4** | **Number of Grooming Behaviors** | | |
|---|---|---|---|
| | | Average (count) | Standard Error |
| Vehicle (+/+) | | 9. 5 | 0.8 |
| Vehicle (j/j) | | 21. 8 | 5.3 |
| Elto (j/j) | | 8.5 | 1 |
| 8OH-DPAT(j/j) | | 5.5 | 0.8 |

As shown in Tables 2 to 4 and FIGs. 1 to 3, administration of eltoprazine improved the spontaneous activity amount, the number of rearing behaviors, and the number of grooming behaviors of Gunn rats to levels comparable to those of normal rats. In contrast, administration of 8OH-DPAT (5-HT_{1A} receptor agonist) improved the spontaneous activity amount and the number of grooming behaviors to levels comparable to those of normal rats, but resulted in abnormal number of rearing behaviors.

### (Example 3)

### (Open Field Test)

Eltoprazine (E1357, TCI), which is one of the 5-HT_{1A/1B} receptor agonists, was dissolved in an arbitrary solvent. 0.3 mg/kg of eltoprazine was subcutaneously administered to jaundice model rats (Gunn rats) at 9 to 10 weeks of age. Further, as control groups, only a solvent was subcutaneously administered to Gunn rats and Wistar rats at 9 to 10 weeks of age. Further, as a comparison group, CGS12066A (Sigma-Aldrich Co. LLC), which is one of 5-HT_{1B} receptor agonists, was dissolved in an arbitrary solvent, and 1 mg/kg thereof was subcutaneously administered to Gunn rats. After placing each rat in its home cage for 60 minutes, an open field test was conducted for 60 minutes using the Scanet MV-40 (Melquest Ltd.). The spontaneous activity amount and the number of rearing behaviors for each rat were measured by counting how many times the rat crossed the center evenly spaced. The number of grooming behaviors was counted by visual inspection. The results are shown in Tables 5 to 7 and FIGs. 4 to 6.

Table 5 and FIG. 4 show a spontaneous activity amount of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of CGS12066A (CGS(j/j)). In the figure, ** indicates p < 0.01 (Dunnett's test comparing each group with the Wistar rats administered only with a solvent).

**[Table 5]**

| **Table 5** | **Spontaneous Activity Amount** | | |
|---|---|---|---|
| | | Average (count) | Standard Error |
| Vehicle(+/+) | | 5920 | 633 |
| Vehicle (j/j) | | 11727 | 2102 |
| Elto(j/j) | | 4640 | 522 |
| CGS(j/j) | | 4030 | 101 |

Table 6 and FIG. 5 show the number of rearing behaviors of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of CGS12066A (CGS(j/j)). In the figure, ** indicates p < 0.01 (Dunnett's test comparing each group with the Wistar rats administered only with a solvent).

**[Table 6]**

| **Table 6** | **Number of Rearing Behaviors** | | |
|---|---|---|---|
| | | Average (count) | Standard Error |
| Vehicle (+/+) | | 72,3 | 8. 7 |
| Vehicle(j/j) | | 119.1 | 11.5 |
| Elto(j/j) | | 61. 5 | 9.6 |
| CGS(j/j) | | 47 | 9.5 |

Table 7 and FIG. 6 show the number of grooming behaviors of the following groups: Wistar rats administered only with a solvent (Vehicle(+/+)), Gunn rats administered only with a solvent (Vehicle(j/j)), Gunn rats administered with 0.3 mg/kg of eltoprazine (Elto(j/j)), and Gunn rats administered with 1 mg/kg of CGS12066A (CGS(j/j)). In the figure, * indicates p < 0.05 (Dunnett's test comparing each group with the Wistar rats administered only with a solvent).

**[Table 7]**

| **Table7** | **Number of Grooming Behaviors** | | |
|---|---|---|---|
| | | Average (count) | Standard Error |
| Vehicle (+/+) | | 9. 5 | 0.8 |
| Vehicle (j/j) | | 21.8 | 5.3 |
| Elto (j/j) | | 8.5 | 1 |
| CGS(j/j) | | 19. 6 | 6.9 |

As shown in Tables 5 to 7 and FIGs. 4 to 6, administration of eltoprazine improved the spontaneous activity amount, the number of rearing behaviors, and the number of grooming behaviors of Gunn rats to levels comparable to those of normal rats. In contrast, administration of CGS12006A (5-HT_{1B} receptor agonist) improved the spontaneous activity amount and the number of rearing behaviors to levels comparable to those of normal rats, but did not improve the number of grooming behaviors.

### (Example 4)

### (Social Interaction Test)

Eltoprazine (E1357, TCI), which is one of 5-HT_{1A/1B} receptor agonists, was dissolved in a physiological saline solution. Only a solvent, 0.3 mg/kg of eltoprazine, or 1 mg/kg of eltoprazine was administered subcutaneously to Gunn rats at 9 to 10 weeks of age. For control, only a solvent was administered to Wistar rats as normal rats. One hour after the administration, two rats of the same type (same drug, same dose, same genetic type) were placed together in a single cage, and their behaviors were video-recorded from above for 10 minutes. The recorded video was visually analyzed, and the number of behaviors of following the partner, the attacking behavior, and the duration of sniffing or grooming the partner were measured.

Table 8 and FIG. 7 show the number of behaviors of following the partner in the following groups: Wistar rats administered only with a solvent (Vehicle(+/+)), Gunn rats administered only with a solvent (Vehicle(j/j)), Gunn rats administered with 0.3 mg/kg of eltoprazine (Elto 0.3 mg(j/j)), and Gunn rats administered with 1 mg/kg of eltoprazine (Elto 1 mg(j/j)). In the figure, *** indicates p < 0.001 (Dunnett's test comparing each group with the Wistar rats administered only with a solvent).

**[Table 8]**

| **Table 8** | **Following Behavior** | | |
|---|---|---|---|
| | | Average (count) | Standard Error |
| Vehicle (+/+) | | 3.5 | 0. 73 |
| Vehicle (j/j) | | 15.3 | 1.57 |
| Elto 0.3mg(j/j) | | 2.16 | 0. 65 |
| Elto 1mg (j/j) | | 3. 83 | 0. 79 |

Administration of eltoprazine reduced the following behavior, which is one of the antagonistic behaviors, to the levels comparable to those of normal rats.

Table 9 and FIG. 8 show the number of attacking behaviors toward the partner in the following groups: Wistar rats administered only with a solvent (Vehicle(+/+)), Gunn rats administered only with a solvent (Vehicle(j/j)), Gunn rats administered with 0.3 mg/kg of eltoprazine (Elto 0.3 mg(j/j)), and Gunn rats administered with 1 mg/kg of eltoprazine (Elto 1 mg(j/j)). In the figure, *** indicates p < 0.001 (Dunnett's test comparing each group with the Wistar rats administered only with a solvent).

**[Table 9]**

| **Table 9** | **Attacking Behavior** | | |
|---|---|---|---|
| | | Average (count) | Standard Error |
| Vehicle (+/+) | | 1. 28 | 0.63 |
| Vehicle (j/j) | | 28 | 2. 35 |
| Elto 0. 3mg(j/j) | | 2 | 1. 43 |
| Elto 1mg(j/j) | | 0.16 | 0.16 |

Administration of eltoprazine reduced the attacking behavior, which is one of the antagonistic behaviors, to the levels comparable to those of normal rats.

Table 10 and FIG. 9 show the duration (seconds) of the behavior of sniffing or grooming the partner in the following groups: Wistar rats administered only with a solvent (Vehicle(+/+)), Gunn rats administered only with a solvent (Vehicle(j/j)), Gunn rats administered with 0.3 mg/kg of eltoprazine (Elto 0.3 mg(j/j)), and Gunn rats administered with 1 mg/kg of eltoprazine (Elto 1 mg(j/j)). In the figure, *** indicates p < 0.001 (Dunnett's test comparing each group with the Wistar rats administered only with a solvent).

**[Table 10]**

| **Table 1 0** | **Sniffing Behavior/Grooming Behavior** | | |
|---|---|---|---|
| | | Average (count) | Standard Error |
| Vehicle (+/+) | | 66. 7 | 6. 59 |
| Vehicle (j/j) | | 11 | 1. 08 |
| Elto 0.3mg(j/j) | | 52 | 9.71 |
| Elto 1mg(j/j) | | 41. 5 | 14 |

Administration of eltoprazine increased the duration of the behavior of sniffing or grooming the partner, which is one of the affiliative behaviors, to the normal level.

### (Example 5)

### (PPI test)

Eltoprazine (E1357, TCI), which is one of 5-HT_{1A/1B} receptor agonists, was dissolved in a physiological saline solution. Only a solvent, 0.3 mg/kg of eltoprazine, or 1 mg/kg of eltoprazine was administered subcutaneously to Gunn rats at 9 to 10 weeks of age. For control, only a solvent was administered to Wistar rats as normal rats. One hour after the administration, a startle response prepulse inhibition (PPI) test was performed. The PPI test was conducted using the SR-LAB-Startle Response System (San Diego Institute, USA). Each rat subjected to the measurement was placed in a measurement chamber and acclimated with a 65 dB background white noise for 5 minutes before the start of stimulation. The startle response to a 120 dB auditory stimulation was measured using a piezoelectric sensor attached to the bottom of the chamber. The test session consisted of a total of 50 randomized trials, including no-stimulus (white noise only), 20 ms of 120 dB pulse stimulation, and stimulation with 20 ms of 70 dB or 80 dB prepulse 100 ms before the 120 dB pulse. The interval between the trials was 20 to 60 seconds (average: 40 seconds). The percentage PPI was calculated using the following formula: 100 - [(startle response to prepulse-pulse stimulation trial - no-stimulation trial) / (pulse-alone trial - no-stimulation trial) × 100].

Table 11 and FIG. 10 show the results of percentage PPI in response to 80 dB prepulse in the following groups: Wistar rats administered only with a solvent (Vehicle(+/+)), Gunn rats administered only with a solvent (Vehicle(j/j)), and Gunn rats administered with 0.3 mg/kg of eltoprazine (Elto 0.3mg(j/j)). In the figure, ** indicates p < 0.01 and *** indicates p < 0.001 (Dunnett's test comparing each group with the Wistar rats administered only with a solvent).

**[Table 11]**

| **Table 1 1** | **Percentage PPI** | |
|---|---|---|
| | Average (%) | Standard Error |
| Vehicle (+/+) | 82.8 | 1.7 |
| Vehicle (j/j) | 72. 6 | 8.3 |
| Elto 0. 3mg(j/j) | 63.6 | 4.9 |

Administration of eltoprazine showed a tendency to improve the percentage PPI, which is one of the indicators used to assess cognitive function.

### (Example 6)

### (Open Field Test)

The 5-HT_{1A/1B} receptor agonist described in the present specification is dissolved in an arbitrary solvent. 0.3 mg/kg of the 5-HT_{1A/1B} receptor agonist is subcutaneously administered to jaundice model rats (Gunn rats) at 9 to 10 weeks of age. Further, as control groups, only a solvent is subcutaneously administered to Gunn rats and Wistar rats at 9 to 10 weeks of age. As necessary, as a comparison group, 8OH-DPAT (0415005, FUJIFILM Wako Pure Chemical Corporation), which is one of 5-HT_{1A} receptor agonists, is dissolved in an arbitrary solvent, and 1 mg/kg thereof is subcutaneously administered to Gunn rats. After placing each rat in its home cage for 60 minutes, an open field test is conducted for 60 minutes using, for example, the Scanet MV-40 (Melquest Ltd.). The spontaneous activity amount and the number of rearing behaviors for each rat are measured by counting how many times the rat crossed the center evenly spaced. The number of grooming behaviors is counted by visual inspection.

### (Example 7)

### (Open Field Test)

The 5-HT_{1A/1B} receptor agonist described in the present specification is dissolved in an arbitrary solvent. 0.3 mg/kg of the 5-HT_{1A/1B} receptor agonist is subcutaneously administered to jaundice model rats (Gunn rats) at 9 to 10 weeks of age. Further, as control groups, only a solvent is subcutaneously administered to Gunn rats and Wistar rats at 9 to 10 weeks of age. As necessary, as a comparison group, CGS12066A (Sigma-Aldrich Co. LLC), which is one of 5-HT_{1B} receptor agonists, is dissolved in an arbitrary solvent, and 1 mg/kg thereof is subcutaneously administered to Gunn rats. After placing each rat in its home cage for 60 minutes, an open field test is conducted for 60 minutes using, for example, the Scanet MV-40 (Melquest Ltd.). The spontaneous activity amount and the number of rearing behaviors for each rat are measured by counting how many times the rat crossed the center evenly spaced. The number of grooming behaviors is counted by visual inspection.

### (Example 8)

### (Social Interaction Test)

The 5-HT_{1A/1B} receptor agonist described in the present specification is dissolved in a physiological saline solution. Only a solvent, 0.3 mg/kg of the 5-HT_{1A/1B} receptor agonist, or 1 mg/kg of the 5-HT_{1A/1B} receptor agonist is administered subcutaneously to Gunn rats at 9 to 10 weeks of age. As a control, only a solvent is administered to Wistar rats, which are normal rats. One hour after the administration, two rats of the same type (same drug, same dose, same genetic type) are placed together in a single cage, and their behaviors were video-recorded from above for 10 minutes. The recorded video data is visually analyzed, and the number of behaviors of following the partner, the attacking behavior, and the duration of sniffing or grooming the partner are measured.

### (Example 9)

### (PPI test)

The 5-HT_{1A/1B} receptor agonist described in the present specification is dissolved in a physiological saline solution. Only a solvent, 0.3 mg/kg of the 5-HT_{1A/1B} receptor agonist, or 1 mg/kg of the 5-HT_{1A/1B} receptor agonist is administered subcutaneously to Gunn rats at 9 to 10 weeks of age. As a control, only a solvent is administered to Wistar rats, which are normal rats. One hour after the administration, a startle response prepulse inhibition (PPI) test is performed. The PPI test is conducted using, for example, the SR-LAB-Startle Response System (San Diego Institute, USA). Each rat subjected to the measurement is placed in a measurement chamber and acclimated with a 65 dB background white noise for 5 minutes before the start of stimulation. The startle response to a 120 dB auditory stimulation is measured using a piezoelectric sensor attached to the bottom of the chamber. The test session consists of a total of 50 randomized trials, including no-stimulus (white noise only), 20 ms of 120 dB pulse stimulation, and stimulation with 20 ms of 70 dB or 80 dB prepulse 100 ms before the 120 dB pulse. The interval between the trials is 20 to 60 seconds (average: 40 seconds). The percentage PPI is calculated using the following formula: 100 - [(startle response to prepulse-pulse stimulation trial - no-stimulation trial) / (pulse-alone trial - no-stimulation trial) × 100].

## Claims

1. A pharmaceutical composition for use in treating, preventing, or managing a bilirubin-induced neurological dysfunction, a psychiatric disorder associated with jaundice, or a psychiatric disorder caused by bilirubin UDP-glucuronosyltransferase gene mutation, wherein the pharmaceutical composition comprises a 5-HT_{1A/1B} receptor agonist.

2. A pharmaceutical composition for use in treating, preventing, or managing a bilirubin-induced neurological dysfunction in a subject, wherein the pharmaceutical composition comprises a 5-HT_{1A/1B} receptor agonist, wherein the subject has a high bilirubin level in his/her biological sample compared to a bilirubin level of a healthy subject, or the subject has jaundice.

3. A pharmaceutical composition for use in treating, preventing, or managing a bilirubin-induced neurological dysfunction in a subject, wherein the pharmaceutical composition comprises a 5-HT_{1A/1B} receptor agonist, wherein the subject has a bilirubin UDP-glucuronosyltransferase gene mutation.

4. A pharmaceutical composition for use in a method for treating, preventing, or managing a bilirubin-induced neurological dysfunction in a subject, wherein the pharmaceutical composition comprises a 5-HT_{1A/1B} receptor agonist,
wherein the method comprises steps of:
(a) obtaining a biological sample from the subject;
(b) measuring a bilirubin level in the biological sample obtained in the step (a); and
(c) administering to the subject a 5-HT_{1A/1B} receptor agonist when the bilirubin level measured in the step (b) is higher than a bilirubin level of a healthy subject, or when it is determined that the subject has jaundice based on the bilirubin level measured in the step (b).

5. A pharmaceutical composition for use in a method for treating, preventing, or managing a bilirubin-induced neurological dysfunction in a subject, wherein the pharmaceutical composition comprises a 5-HT_{1A/1B} receptor agonist,
wherein the method comprises steps of:
(a) determining whether the subject has a bilirubin UDP-glucuronosyltransferase gene mutation; and
(b) administering to the subject a 5-HT_{1A/1B} receptor agonist when it is determined that the subject has a bilirubin UDP-glucuronosyltransferase gene mutation in the step (a).

6. A pharmaceutical composition for use in treating, preventing or managing a psychiatric disorder in a subject, wherein the pharmaceutical composition comprises a 5-HT_{1A/1B} receptor agonist, wherein the subject has a high bilirubin level in his/her biological sample compared to a bilirubin level of a healthy subject, or the subject has jaundice.

7. A pharmaceutical composition for use in treating, preventing, or managing a psychiatric disorder in a subject, wherein the pharmaceutical composition comprises a 5-HT_{1A/1B} receptor agonist, wherein the subject has a bilirubin UDP-glucuronosyltransferase gene mutation.

8. A pharmaceutical composition for use in a method for treating, preventing, or managing a psychiatric disorder in a subject, wherein the pharmaceutical composition comprises a 5-HT_{1A/1B} receptor agonist,
wherein the method comprises steps of:
(a) obtaining a biological sample from the subject;
(b) measuring a bilirubin level in the biological sample obtained in the step (a); and
(c) administering to the subject a 5-HT_{1A/1B} receptor agonist when the bilirubin level measured in the step (b) is higher than a bilirubin level of a healthy subject, or when it is determined that the subject has jaundice based on the bilirubin level measured in the step (b).

9. A pharmaceutical composition for use in a method for treating, preventing, or managing a psychiatric disorder in a subject, wherein the pharmaceutical composition comprises a 5-HT_{1A/1B} receptor agonist,
wherein the method comprises steps of:
(a) determining whether the subject has a bilirubin UDP-glucuronosyltransferase gene mutation; and
(b) administering to the subject a 5-HT_{1A/1B} receptor agonist when it is determined that the subject has a bilirubin UDP-glucuronosyltransferase gene mutation in the step (a).

10. The pharmaceutical composition according to any one of claims 1 to 9, wherein the 5-HT_{1A/1B} receptor agonist is a compound of the following formula (I), or a pharmaceutically acceptable salt, solvate, hydrate, stereoisomer, tautomer, or a deuterium-labeled compound thereof, 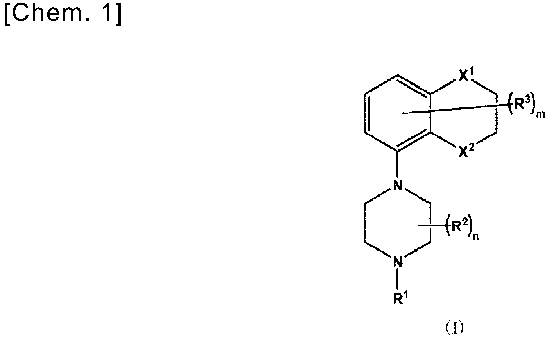
wherein R¹ is hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
R² and R³ are each independently, at each occurrence, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
X¹ and X² are each independently oxygen, sulfur, or -CR^{a}R^{b}; R^{a} and R^{b} are each independently hydrogen, halogen, hydroxy, amino, C₁₋₆ alkyl, C₂₋₆ alkenyl, C₂₋₆ alkynyl, C₁₋₆ hydroxyalkyl, or C₁₋₆ aminoalkyl;
n is an integer of 0 to 4; and
m is an integer of 0 to 5.

11. The pharmaceutical composition according to claim 10, wherein, in the compound of formula (I), X¹ and X² are oxygen.

12. The pharmaceutical composition according to claim 10, wherein, in the compound of formula (I), R¹ is hydrogen.

13. The pharmaceutical composition according to claim 10, wherein, in the compound of formula (I), n is 0 and m is 0.

14. The pharmaceutical composition according to claim 10, wherein, in the compound of formula (I), R¹ is hydrogen, n is 0, and m is 0.

15. The pharmaceutical composition according to claim 10, wherein the compound of formula (I) is eltoprazine.

16. The pharmaceutical composition according to any one of claims 1 to 9, wherein the psychiatric disorder is schizophrenia or attention deficit hyperactivity disorder.

17. A kit for use in treating, preventing, or managing a bilirubin-induced neurological dysfunction, a psychiatric disorder associated with jaundice, or a psychiatric disorder caused by bilirubin UDP-glucuronosyltransferase gene mutation, wherein the kit comprises a 5-HT_{1A/1B} receptor agonist.

18. The kit according to claim 17, further comprising a reagent for measuring a bilirubin level in a biological sample and/or a reagent for measuring bilirubin UDP-glucuronosyltransferase gene mutation.
